# EUROPEAN PATENT APPLICATION

(11) **EP 4 509 537 A1**
(43) Date of publication of application: **19.02.2025**
(21) Application number: 23788537.1
(22) Date of filing: 07.04.2023
(51) Int. Cl.: C08F 216/06, C08F 4/70

(54) **METHOD FOR PRODUCING CHEMICALLY RECYCLABLE POLY(VINYL ALCOHOL) COPOLYMER THROUGH RING-OPENING METATHESIS POLYMERIZATION OF CYCLOHEXENE DERIVATIVES**

(30) Priority: 11.04.2022 KR 20220044543
(71) Applicant: Korea Advanced Institute of Science and Technology, Daejeon 34141 (KR)
(72) Inventor: HONG, Soon Hyeok, Daejeon 34141 (KR); CHOI, Kyungmin, Daejeon 34141 (KR)
(74) Representative: Mewburn Ellis LLP
(86) International application number: PCT/KR2023/004713
(87) International publication number: WO 2023/200186

(57) **Abstract**

The present invention relates to a method for producing a chemically recyclable polyvinyl alcohol copolymer through ring-opening metathesis polymerization of cyclohexene derivatives, wherein the ring strain energy of cyclohexene monomers is increased using trans-fused cyclic carbonate groups to perform ring-opening metathesis polymerization (ROMP) while efficiently controlling same to produce a polyvinyl alcohol copolymer with a well-defined structure of 1,2-diol per six carbon atoms and exhibiting high hydrolytic stability, excellent oxygen protective properties, and processability. In addition, the polyvinyl alcohol copolymer can be chemically recycled into 1,2-diol containing cyclohexene through the ring-closing metathesis of the polyvinyl alcohol polymer, and useful compounds, including industrially useful α,ω-dialdehyde compounds, can be produced by chemically cleaving 1,2-diol groups.

## Description

### [Technical Field]

The present invention relates to a method for producing a chemically recyclable polyvinyl alcohol copolymer through ring-opening metathesis polymerization of cyclohexene derivatives, wherein the ring strain energy of cyclohexene monomers is increased using trans-fused cyclic carbonate groups to perform ring-opening metathesis polymerization (ROMP) while efficiently controlling same to produce a polyvinyl alcohol copolymer with a well-defined structure of 1,2-diol per six carbon atoms and exhibiting high hydrolytic stability, excellent oxygen protective properties, and processability.

### [Related Art]

Every year, more than 380 million tons of polymers are produced, mostly from finite fossil resources (>99%), and 302 million tons of polymer wastes are discarded (Geyer, R. et al., Sci. Adv. 2017, 3, e1700782; The global bio-based polymer market in 2019 - A revised view). The polymer wastes have been exponentially accumulated because of their high durability, causing severe environmental issues. Therefore, the recycling of polymers is critical for a sustainable future. Chemically recyclable polymers have recently been highlighted as an ideal solution for chemical circularity (Sathe, D. et al., Nat. Chem. 2021, 13, 743-750; Chazovachii, P. T. et al. 2021, 12, 4524; Shi, C. et al., Chem 2021, 7, 2896-2912). Chemical recycling involves the depolymerization of polymeric materials into small molecules by chemical treatment. Produced molecules can serve as a monomer to be re-polymerized to the starting polymer (a closed-loop cycle) or can be reused as a chemical feedstock for other applications (an open-loop cycle) (Chazovachii, P. T. et al., Nat. Commun. Chem. 2021, 12, 4524; Ha, K. H. et al., Mater. Des. 2012, 35, 310-317; Larrain, M. et al. 2020, 270, 122442). Ring-opening metathesis polymerization (ROMP) is an attractive choice to develop chemically recyclable polymers with hydrolytic and thermal stability (Sathe, D. et al., Nat. Chem. 2021, 13, 743-750). Olefin metathesis is a reversible reaction, therefore, in theory, the ROMP polymers can be depolymerized back to their monomers via ring-closing metathesis (RCM) reaction for closed-loop recycling by controlling reaction equilibrium (Sathe, D. et al., Nat. Chem. 2021, 13, 743-750; Ofstead, E. A. et al. 1972, 154, 21-34; Schrock, R. R. et al., Macromolecules 1989, 22, 3191-3200; Tuba, R. et al., Polym. Chem. 2013, 4; Tuba, R. et al. Chem. Eng. 2016, 4, 6090-6094; Liu, H. et al., ACS Macro Lett. 2018, 7, 933-937; Neary, W. J. et al. Chem. Soc. 2020, 142, 1186-1189). However, because the control of equilibrium between ROMP and ring-closing metathesis depolymerization (RCMD) is governed by thermodynamics, commonly used ROMP monomers with high ring-strain energies (RSEs), such as norbornene and cyclooctene derivatives, are not suitable to be recycled by RCMD (Sathe, D. et al., Nat. Chem. Letters, 2021, 13, 743-750; Tuba, R. et al. Chem. 2013, 4; Monfette, S. et al. Rev. 2009, 109, 3783-3816).

ROMP of cyclohexene, which has the least ring strain energy (RSE, 2.50 kcal/mol) among small cyclic olefins, is a long-standing challenge in metathesis (Sathe, D. et al., Nat. Chem. 2021, 13, 743-750; Neary, W. J. et al., ACS Macro Lett. 2019, 8, 46-56; Patton, P. A. et al., Macromolecules 1986, 19, 1266-1268; Mol, J. C. et al., Elsevier Science & Technology: 1997; Choi, T. L. et al., J. Am. Chem. Soc. 2001, 123, 10417-10418; Hejl, A. S. et al., Macromolecules 2005, 38, 7214-7218; Park, H. et al., J. Am. Chem. Soc. 2012, 134, 7270-7273; Hlil, A. R. et al., J. Polym. Sci., Part A: Polym. Chem. 2017, 55, 3137-3145). The homopolymerization of cyclohexene was only reported by the McCarthy Group, which demonstrated a low conversion (12%) to oligomers with 2-6 degrees of polymerization (DP) using WCl₆/SnMe₄ catalyst at -77°C (Patton, P. A. et al., Macromolecules 1986, 19, 1266-1268). Despite the hurdle, cyclohexene derivatives could serve as an attractive monomer because cyclohexene derivatives can be readily obtained from the reduction of aromatic compounds, diversely functionalizable with enriched synthetic pathways, and facilely recyclable by RCMD from the thermodynamic advantage due to their low RSEs. Recently, the Chen group reported that the ROMP polymerization of a bridged γ-butyrolactone-based cyclohexene derivative monomer (6-oxabicyclo[3.2.1]oct-3-en-7-one) is possible (Shi, C. et al., J. Am. Chem. Soc. 2022, 144, 2264-2275).

In order to develop new functional polymers that can be chemically recycled by utilizing various cyclohexene derivatives as monomers, which are difficult to apply the conventional ring-opening metathesis polymerization methods, cyclohexene derivatives with appropriate functional groups were elaborately designed. Trans-fused 5-membered cyclic carbonates functionalized with 6-membered rings have been reported as compounds with relatively high RSE (Tezuka, K. et al., Polym. J. 2013, 45, 1183-1187; Guerin, W. et al., Macromolecules 2014, 47, 4230-4235; Diallo, A. K. et al., Polym. Chem. 2015, 6, 1961-1971). High RSE can be caused by distortion due to opposing C-O bonds in the same plane by the carbonate group. The utilization of carbonate groups to provide oxygen-containing functional groups used to modulate RSE can have several advantages. Carbonate groups can be easily deprotected by hydrolysis under acidic or basic conditions to generate 1,2-dihydroxy groups. In addition, cyclic carbonates can undergo a variety of reactions with amines, alcohols, and other nucleophiles to form a variety of functional groups, including cross-linking functional groups, to tailor material properties to suit their purpose. Finally, cyclic carbonate-containing polymers can be used as polymeric electrolytes in energy storage applications.

To address the above problems, the inventors of the present invention have used trans-fused cyclic carbonate groups to increase the ring strain energy of cyclohexene monomers by performing ring-opening metathesis polymerization reactions under efficient control, resulting in producing polyvinylalcohol copolymers with a well-defined structure of 1,2-diols per six carbons and high hydrolytic stability, good oxygen protection properties and processability, and found that the polymer can be chemically recycled to 1,2-diol-containing cyclohexenes by ring-closing metathesis polymerization demonstrating closed-loop recycling, or chemically degraded by cleavage of the 1,2-diol group to produce industrially useful substances such as α,ω-dialdehyde or dicarboxylic acid compounds, and have completed the present invention.

### [Detailed Description of the Invention]

### [Technical Problem]

It is an object of the present invention to provide a method for efficiently controlling a ring-opening metathesis polymerization by increasing the ring strain energy of a cyclohexene monomer.

Another object of the present invention is to provide a polyvinyl alcohol copolymer having a well-defined structure of 1,2-diol per six carbons with high hydrolytic stability, good oxygen protection properties and processability, which has not been previously reported to prepare.

It is another object of the present invention to provide a method for preparing useful compounds, including industrially useful 1,2-diol-containing cyclohexenes, α,ω-dialdehyde or dicarboxylic acid compounds, by chemically recycling of the developed polymers.

### [Technical Solution]

To achieve the above objectives, the present invention provides a method for preparing a polyvinyl alcohol copolymer and a polycarbonate, comprising (a) subjecting a cyclohexene compound with a carbonate protecting group to a ring-opening metathesis polymerization (ROMP) in the presence of a catalyst to obtain a polycarbonate of Formula 1; and (b) subjecting the compound of Formula 1 to carbonate deprotection to obtain a polyvinyl alcohol copolymer of Formula 4: 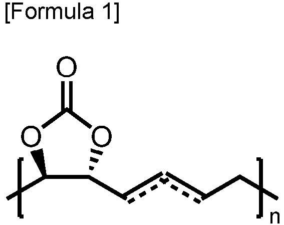 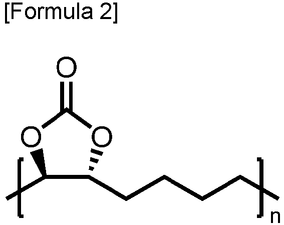 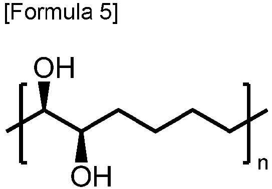 wherein n is an integer of 1 to 100,000.

The present invention also provides a polyvinyl alcohol copolymer represented by Formula 4 or Formula 5: 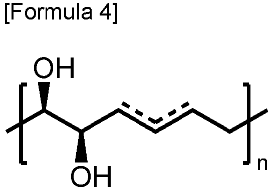 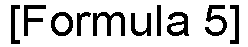 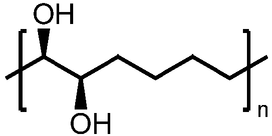 wherein n is an integer of 1 to 100,000.

The present invention also provides a method for recycling a cyclohexene monomer, comprising (a) subjecting a polyvinyl alcohol copolymer of Formula 4 to a ring-closing metathesis (RCM) to obtain a compound of Formula 6; and (b) subjecting the copolymer of Formula 6 to carbonate protection to obtain a cyclohexene monomer: 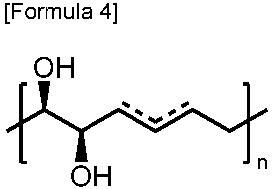 wherein n is an integer of 1 to 100,000.

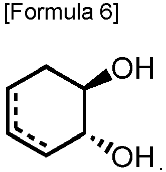

The invention also provides a method of producing a useful organic material, comprising performing a diol oxidation reaction of a polyvinyl alcohol copolymer of Formula 5 to obtain a carboxylic acid of Formula 7, an aldehyde of Formula 8, or an alcohol of Formula 9: 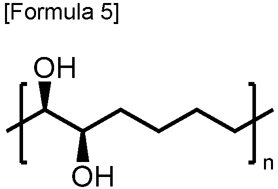 wherein n is an integer of 1 to 100,000.

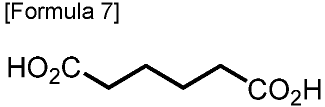

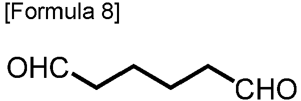

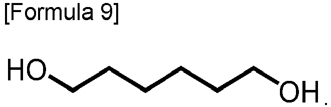

The present invention also provides a polymeric electrolyte comprising the polyvinyl alcohol copolymers.

### [Effect of the Invention]

According to the present invention, the ROMP of the resulting carbonate monomer can be efficiently controlled by increasing the ethenolysis ring strain energy of cyclohexene diol. The obtained carbonate polymer can be deprotected to produce a hydroxyl-rich polymer with a well-defined structure of 1,2-diol per six carbons, which has high hydrolytic stability, good oxygen protection properties and processability. The resulting polymer can be chemically recycled to 1,2-diol-containing cyclohexenes by a ring-closing metathesis demonstrating closed-loop recycling. Additionally, the 1,2-diol polymer can be chemically degraded by cleavage of the 1,2-diol group of the polymer to produce industrially useful α,ω-dialdehyde or dicarboxylic acid compounds.

### [Brief Description of the Drawing]

FIG. 1 is a diagram illustrating the ring-opening metathesis homopolymerization of cyclohexene.
FIG. 2 is a diagram showing (a) the definition of ethenolysis ring strain energy (ERSE); and (b) the results of DFT calculations of ERSE values for candidate cyclohexene monomers.
FIG. 3 is a diagram showing the Newman projection of the carbonate monomer on the bridgehead carbons. Structural information was obtained by DFT calculations at the B3YLP/6-31G(d,p) level. Structural information obtained by single crystal XRD is shown in parentheses.
FIG. 4 is a diagram depicting (a) ¹H NMR spectra for P3 and P5 with assigned structures; (b) conversion rates of monomers 3 and 5 versus polymerization time; and (c) molecular weights of the polymers versus initial monomer to initiator ratio (molecular weight of P3 and P5 versus conversion rate using Ru1 at 10°C, initial monomer to initiator ratio is 200:1).
FIG. 5 is a graph showing the structure of the H-T, H-H, T-T conjugation, and ¹H-¹H COSY spectra of P5.
FIG. 6 is a diagram showing the synthesis and thermal properties of the postfunctionalized polymers of P3 and P5.
FIG. 7 shows strain stress curves for P_{D}3, P_{H}3, and P_{HD}3 films (P_{D}3: red, P_{H}3: black, and P_{HD}3: blue).
FIG. 8 is a diagram illustrating the recycling and upcycling of PVA copolymers.
FIG. 9 shows the differential scanning calorimetry curves for a) P3, b) P_{D}3, c) P_{H}3, d) P_{HD}3, e) P5, f) P_{D}5, g) P_{H}5, and h) P_{HD}5.
FIG. 10 shows the thermogravimetric analysis curves of a) P3, b) P_{D}3, c) P_{H}3, d) P_{HD} 3, e) P5, f) P_{D}5, g) P_{H}5, and h) P_{HD}5.
FIG. 11 shows the ¹H NMR spectra of (a) P_{D}3 after exposure to P_{D}3 (black), acidic (red) and basic (blue) conditions and (b) P_{D}5 after exposure to P_{D}5 (black), acidic (red) and basic (blue) conditions.
FIG. 12 shows a) an overlay of GPC traces of P_{D}3 after exposure to P_{D}3 (black) and an aqueous solution of HCl (pH 3) (red) for 18 hours; b) an overlay of GPC traces of P_{D}3 after exposure to P_{D} 3 (black) and an aqueous solution of 1 M NaOH (pH 14) (blue) for 18 hours (monomer to initiator ratio of 1000:1).
FIG. 13 shows a) an overlay of GPC traces of P_{D}5 after exposure to P_{D}5 (black) and an aqueous solution of HCl (pH 2) (red) for 18 hours; b) an overlay of GPC traces of P_{D}5 after exposure to P_{D}5 (black) and an aqueous solution of 1 M NaOH (pH 14) (blue) for 18 hours (monomer to initiator ratio of 1000:1).
FIG. 14 is a drawing showing the IR spectra of P_{HD}3 after exposure to P_{HD}3 (black), 1 M *aq.* HCl (pH 1) (red) and 1 M aq. NaOH (pH 14) (blue) for 18 hours.
FIG. 15 is a diagram showing the IR spectra of P_{HD}5 after exposure to P_{HD}5 (black), 1 M *aq.* HCl (pH 1) (red) and 1 M aq. NaOH (pH 14) (blue) for 18 hours.
FIGs. 16 and 17 are drawings of the oxygen transmission rate (OTR) and water vapor transmission rate (WVTR) of P3.
FIG. 18 and FIG. 19 are drawings of the oxygen transmission rate (OTR) and water vapor transmission rate (WVTR) of P5.
FIG. 20 is a diagram of the oxygen transmission rate (OTR) of P_{D}3.
FIG. 21 is a diagram of the oxygen transmission rate (OTR) of P_{D}5.
FIGs. 22 and 23 are drawings of the oxygen transmission rate (OTR) and water vapor transmission rate (WVTR) of P_{H}3.
FIGs. 24 and 25 are drawings of the oxygen transmission rate (OTR) and water vapor transmission rate (WVTR) of P_{H}5.
FIGs. 26 and 27 are drawings of the oxygen transmission rate (OTR) and water vapor transmission rate (WVTR) of P_{HD}3.
FIG. 28 is a diagram of the ¹H spectrum of P_{HD}3 after decomposition by oxidative decomposition.
FIG. 29 is a diagram of the ¹H spectrum of P_{HD}5 after degradation by oxidative decomposition.
FIGs. 30 to 34 are drawings illustrating ¹H, ¹³C, ¹H-¹H COSY, ¹H-¹³C HSQC NMR and IR spectra of poly(trans-1,4-cyclohexadiene carbonate) (P3).
FIGs. 35 to 39 are drawings illustrating ¹H, ¹³C, ¹H-¹H COSY, ¹H-¹³C HSQC NMR and IR spectra of poly(trans-1,3-cyclohexadiene carbonate) (P5).
FIGs. 40 to 44 are drawings illustrating ¹H, ¹³C, ¹H-¹H COSY, ¹H-¹³C HSQC NMR and IR spectra of poly(trans-1-cyclohexen-4,5-diol) (P_{D}3).
FIGs. 45 to 49 are drawings illustrating ¹H, ¹³C, ¹H-¹H COSY, ¹H-¹³C HSQC NMR and IR spectra of poly(trans-1-cyclohexen-3,4-diol) (P_{D}5).
FIGs. 50 to 54 are drawings illustrating ¹H, ¹³C, ¹H-¹H COSY, ¹H-¹³C HSQC NMR and IR spectra of poly(trans-3,4-carbonate-hexane-1,6-diyl) (P_{H}3).
FIGs. 55 to 59 are drawings illustrating ¹H, ¹³C, ¹H-¹H COSY, ¹H-¹³C HSQC NMR and IR spectra of poly(trans-2,3-carbonate-hexane-1,6-diyl-ran-trans-4,5-carbonate-hexane-1,6-diyl) (P_{H}5).
FIG. 60 is a diagram showing the IR spectrum of poly(trans-3,4-dihydroxyhexane-1,6-diyl) (P_{HD}3).
FIG. 61 is a diagram showing the IR spectrum of poly(trans-2,3-dihydroxyhexane-1,6-diyl-ran-trans-4,5-dihydroxyhexane-1,6-diyl) (P_{HD}5).
FIG. 62 is a diagram showing GPC results of a) P3, b) P_{H}3, and c) P_{D}3 with a monomer to initiator ratio of 1000:1.
FIG. 63 is a diagram showing GPC results of a) P5, b) P_{H}5, and c) P_{D}5 with a monomer to initiator ratio of 1000:1.
FIG. 64 is a diagram showing IR spectra of a) poly(*trans-1*,4-cyclohexadiene carbonate) (P3), b) poly(*trans*-3,4-carbonate-hexane-1,6-diyl) (P_{H}3), *c)* poly(*trans*-1-cyclohexene-4,5-diol) (P_{D}3), and d) poly(*trans*-3,4-dihydroxyhexane-1,6-diyl) (P_{HD}3).
FIG. 65 shows IR spectra of a) poly(*trans*-1,3-cyclohexadiene carbonate) (P5), b) poly(*trans*-2,3-carbonate-hexane-1,6-diyl-ran-trans-4,5-carbonate-hexane-1,6-diyl) (P_{H}5), c) poly(*trans*-1-cyclohexene-3,4-diol) (P_{D}5), and d) poly*(trans*-2,3-dihydroxyhexane-1,6-diyl-ran-trans-4,5-dihydroxyhexane-1,6-diyl) (P_{HD}5).
FIG. 66 is a drawing comparing the ¹H NMR spectrum of P_{D}3 and the ¹H NMR spectra of the monomer (7) and the polymer (P_{D}3) after ring-closing metathesis depolymerization. All spectra were measured in DMSO-d*₆* and spectra ppm was normalized to solvent retention peaks.
FIG. 67 is a drawing comparing the ¹H NMR spectrum of P_{D}5 and the ¹H NMR spectra of the monomer (9) and the polymer (P_{D}5) after ring-closing metathesis depolymerization. All spectra were measured in DMSO-d*₆* and spectra ppm was normalized to solvent retention peaks.

### [Best Mode]

Unless otherwise defined, all technical and scientific terms used herein have the same meaning as commonly understood by a person skilled in the art. In general, the nomenclature used herein is well known and in common use in the art.

The present invention can efficiently perform ring-opening metathesis polymerization (ROMP) by controlling the ring strain energy of six-membered cyclic olefin compounds using trans-fused cyclic carbonate groups. Cyclohexene monomers are rationally designed from cyclohexene diols to increase the ring strain energy. Increasing the ethenolysis ring-strain energy (ERSE) of cyclohexene diol to about 6-10 kcal/mol by converting the 1,2-diol group to a carbonate group can efficiently control the ROMP of the resulting carbonate monomer.

The obtained carbonate polymer can be deprotected to generate a hydroxyl-rich polymer with a well-defined structure of 1,2-diol per six carbons, which was found to have high hydrolytic stability, good oxygen protection properties and processability.

Accordingly, the present invention relates, in one aspect, to a method for the preparation of a polyvinyl alcohol copolymer, comprising the steps of (a) subjecting a cyclohexene compound with a carbonate protecting group to a ring-opening metathesis polymerization (ROMP) in the presence of a catalyst to obtain a compound of Formula 1; and (b) subjecting the compound to carbonate deprotection to obtain a polyvinyl alcohol copolymer of Formula 4.

The present invention relates, in another aspect, to a method for the preparation of a polyvinyl alcohol copolymer, comprising the steps of (a) subjecting a cyclohexene compound with a carbonate protecting group to a ring-opening metathesis polymerization (ROMP) in the presence of a catalyst to obtain a polycarbonate of Formula 1; (b) subjecting a hydrogenation reaction in the presence of a catalyst to obtain a polycarbonate of Formula 2; and (c) deprotecting the carbonate group to obtain a polyvinyl alcohol copolymer of Formula 5.

In another aspect, the present invention relates to a polyvinyl alcohol copolymer represented by Formula 4 or Formula 5. 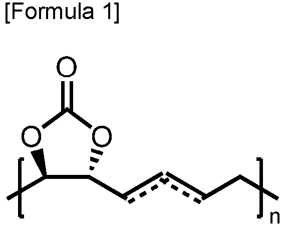 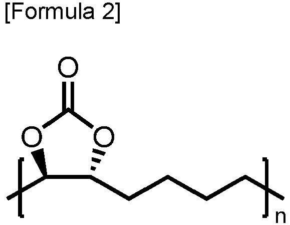 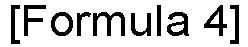 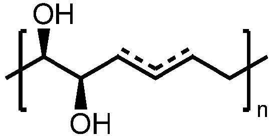 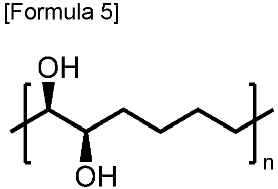 wherein n is an integer of 1 to 100,000.

The present invention is described in detail below.

The present invention relates to a metathesis homopolymerization of cyclohexene derivatives. The polymerization was possible by controlling the ring strain using trans-carbonate fused cyclohexene monomers (RSE = 6-10 kcal/mol). In particular, the resulting cyclohexene-based polymers can be recycled by devising both open-loop and closed-loop recycling strategies through appropriate chemical modifications. Deprotection of the resulting carbonate polymers can produce well-defined PVA-type copolymers with a 1,2-diol structure in the polymer backbone. The polymers obtained by converting the carbonate groups to 1,2-diols are easy to be degraded and recycled. The low ring strain of cyclohexene-1,2-diol allows the resulting PVA-type polymers to be subjected to closed-loop chemical recycling via RCMD. In addition, the 1,2-diol polymers can be degraded by oxidative cleavage of the 1,2-diol on the polymer backbone to yield industrially useful α,ω-dialdehyde and dicarboxylic acid compounds, demonstrating a novel open-loop upcycling strategy for ROMP polymers. Comparing the hydrolysable units such as the previously reported acetals (Fraser, C. et al., Macromolecules 1995, 28, 7256-7261; Debsharma, T. et al., Angew. Chem. Int. Ed. Engl. 2019, 58, 6718-6721; Debsharma, T. et al., Macromolecules 2021, 54, 2720-2728), enol ethers (Feist, J. D. et al., J. Am. Chem. Soc. 2020, 142, 1186-1189; Feist, J. D. et al. 2021, 14, 53-58), silyl ethers (Shieh, P. et al., Nat. Chem. 2019, 11, 1124-1132; Shieh, P. et al, ACS Macro Lett. 2021, 10, 805-810) with the degradable ROMP polymers used for polymer skeleton degradation, the developed 1,2-diol polymers have a polymer skeleton composed of only carbon-carbon bonds and thus have hydrolytic stability, which can be very advantageous for their potential application as oxygen blocking materials.

A method of preparing a polyvinyl alcohol copolymer according to one embodiment of the present invention includes the steps of (a) subjecting a cyclohexene compound with a carbonate protecting group to a ring-opening metathesis polymerization (ROMP) in the presence of a catalyst; and (b) subjecting the compound to carbonate deprotection to obtain a polyvinyl alcohol copolymer.

Furthermore, a method of preparing a polyvinyl alcohol copolymer according to another embodiment of the present invention comprises the steps of (a) subjecting a cyclohexene compound with a carbonate protecting group to a ring-opening metathesis polymerization (ROMP) in the presence of a catalyst; (b) subjecting the compound to a hydrogenation reaction in the presence of a catalyst; and (c) subjecting the compound to carbonate deprotection to obtain a polyvinyl alcohol copolymer.

In the present invention, the cyclohexene compound can be obtained from cyclohexene diols by converting the 1,2-diol group to a carbonate group.

In the present invention, the ethenolysis ring-strain energy of the cyclohexene compound may be at least 1.5 kcal/mol, preferably 1.5 to 30 kcal/mol, more preferably 1.5 to 20 kcal/mol, and especially preferably 1.62 to 10.3 kcal/mol.

In the present invention, the cyclohexene compound to which the carbonate protecting group is bonded may be represented by Formula 3.

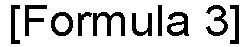

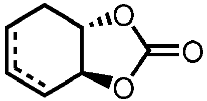

Preferably, the cyclohexene compound may be trans-4,5-cyclohexadiene carbonate of Formula 3-1 or trans-3,4-cyclohexadiene carbonate of Formula 3-2, wherein trans-4,5-cyclohexadiene carbonate has an ERSE of 6.00 kcal/mol and trans-3,4-cyclohexadiene carbonate has an ERSE of 10.3 kcal/mol.

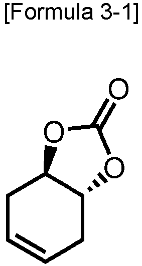

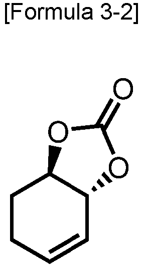

In the present invention, the catalyst in the ring-opening metathesis polymerization (ROMP) may be selected from the following formulae.

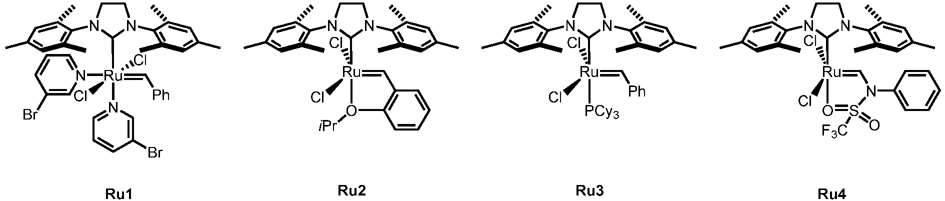

In the present invention, the catalysts in the hydrogenation reactions may be selected from the group consisting of ruthenium pincer complexes such as RuCl₂(PPh₃)₃, Pd/C, Ru-MACHO^{®} (Bis[2-(diphenylphosphino)ethyl]amine}carbonylchlorohydrido ruthenium(II)), rhodium catalysts, such as (cyclohexyl-CAAC)Rh(COD)Cl, BINAP ligand + Rh), and TsNHNH₂ or hydrogen (H₂).

The resulting polymer can be chemically recycled to 1,2-diols containing cyclohexene by a ring-closing metathesis demonstrating closed-loop recycling. It was also found that the 1,2-diol polymer can be chemically degraded by cleavage of the 1,2-diol group of the polymer to prepare useful compounds including industrially useful α,ω-dialdehydes and dicarboxylic acid compounds.

Accordingly, the present invention relates, in another aspect, to a method of recycling cyclohexene monomers comprising the steps of (a) subjecting a polyvinyl alcohol copolymer of Formula 4 to a ring-closing metathesis (RCM) reaction to obtain a compound of Formula 6; and (b) subjecting the compound to carbonate protection to obtain a cyclohexene monomer. 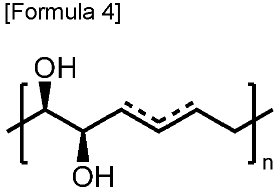 wherein n is an integer of 1 to 100,000.

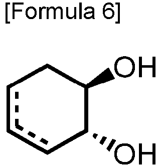

In another aspect, the present invention relates to a method for the preparation of a useful organic substance, comprising the step of performing a diol oxidation reaction of a polyvinyl alcohol copolymer of Formula 5 to obtain a carboxylic acid of Formula 7, an aldehyde of Formula 8, or an alcohol of Formula 9. 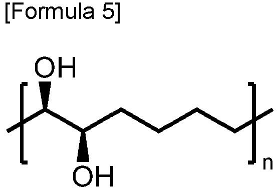 wherein n is an integer of 1 to 100,000.

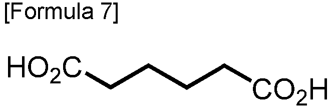

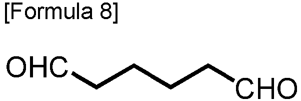

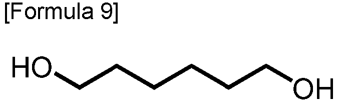

A method for recycling cyclohexene monomers by one preferred embodiment of the present invention comprises (a) subjecting a cyclohexene compound with a carbonate protecting group to a ring-opening metathesis polymerization (ROMP) in the presence of a catalyst; (b) performing a carbonate deprotection to obtain a polyvinyl alcohol copolymer; (c) subjecting the polyvinyl alcohol copolymer to RCM; and (d) performing a carbonate protection to obtain a cyclohexene monomer.

A method for the preparation of a useful organic material by a preferred embodiment of the present invention comprises (a) performing a ring-opening metathesis polymerization (ROMP) of a cyclohexene compound with a carbonate protecting group in the presence of a catalyst; (b) performing a hydrogenation reaction in the presence of a catalyst; (c) performing a carbonate deprotection to obtain a polyvinyl alcohol copolymer; and (d) performing a diol oxidation reaction of the polyvinyl alcohol copolymer to obtain a carboxylic acid, aldehyde, or alcohol.

Another aspect of the present invention relates to a polymeric electrolyte comprising the polyvinyl alcohol copolymers.

The polymer electrolyte may have an ionic conductivity of 1.00×10⁻⁸ or more, preferably 1.00×10⁻⁸ to 9.99×10⁻² Scm⁻¹ at 30 to 80°C when containing 25% electrolyte (bis(trifluoromethane)sulfonimide, LiTFSI). The polymeric electrolyte can be easily prepared as a film, and the resulting film has transparent and flexible properties. If the ionic conductivity is less than 1.00×10⁻⁸, there is a problem that the ionic conductivity is too low to be used as an electrolyte. To further increase the ionic conductivity, it may be difficult to manufacture the film due to the high salt ratio.

Hereinafter, preferred examples of the present invention are described for the purpose of illustrating the present invention, but it is apparent to those skilled in the art that various changes and modifications are possible within the scope of the present invention and the technical idea, and that such changes and modifications fall within the scope of the appended patent claims.

### [Examples]

### Example 1: Regulating the ring strain energies of cyclohexene monomers

Cyclic olefin monomers, elaborately designed to control their RSEs, have been well utilized to perform ROMP or RCMD more efficiently or to enable them. For example, Grubbs and co-workers synthesized poly(cyclopentene-3,5-diol) by using a sacrificial silyl protecting group on cyclopentene-3,5-diol with low strain to form a highly modified cross-linked heterocyclic structure, subjecting ROMP, and subjecting deprotection (Scherman, O. A. et al., Macromolecules 2002, 35, 5366-5371). Yang and coworkers performed an effective ROMP of macrocyclic monomers by generating eight-membered rings with high RSE via a sacrificial silyloxy bridge on a macrocyclic monomer with low RSE (Yu, Z. et al., Angew. Chem. Int. Ed Engl. 2022, 61, e202112526). Recently, Wang and coworkers reported an effective ROMP and RCMD of cyclooctene-based monomers by controlling the monomer RSE. The RSE of the monomers was reduced by fusing trans-cyclobutane to cyclooctene and increased by isomerizing the resulting monomers into highly modified trans isomers (Sathe, D. et al., Nat. Chem. Chem. 2021, 13, 743-750; Chen, H. et al. Int. Ed Engl. 2021, 60, 25493-25498).

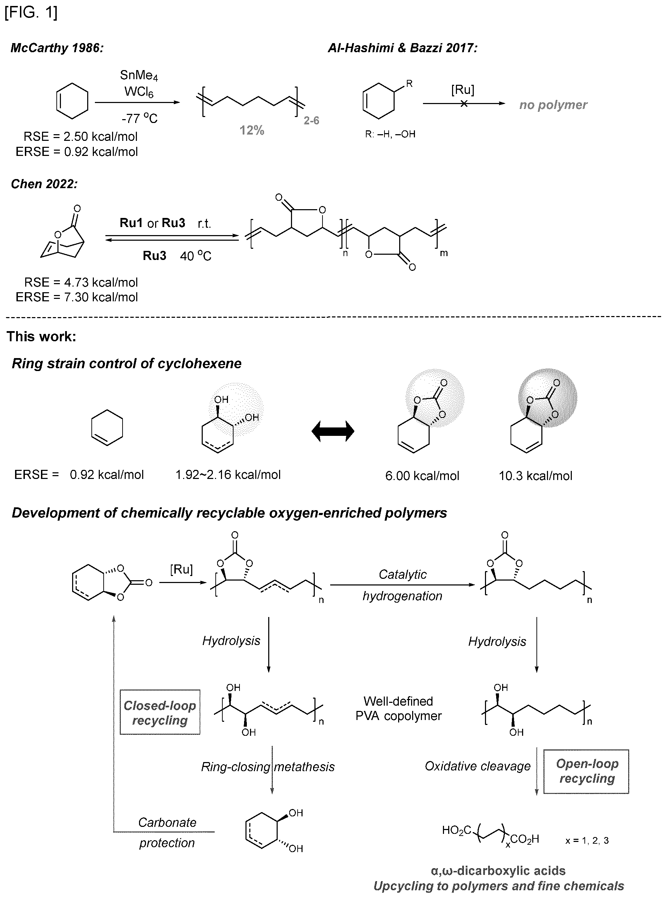

In order to utilize versatile cyclohexene monomers to develop polymers that can be chemically recycled by ROMP, the RSE of various oxygen atom-containing cyclohexene derivatives was first investigated by theoretical methods. Instead of applying the traditionally defined RSE based on the Benson group incremental theory, the enthalpy changes of the ethenolysis reaction, which has often been used to predict monomer reactivity in ROMP (Sathe, D. et al., Nat. Chem. 2021, 13, 743-750; Hejl, A. et al., Macromolecules 2005, 38, 7214-7218; Yarolimek, M. R. et al., ACS Macro Lett. 2021, 10, 760-766), was used to more appropriately model the targeted metathesis reaction (FIG. 1(a)). This enthalpy is referred to as the ethenolysis ring strain energy (ERSE).

As mentioned above, the trans-fused five-membered cyclic carbonate groups are expected to increase the RSE of the parent cyclohexene ring. To confirm the carbonate protection effect on cyclohexene, the ERSE was calculated by density functional theory (DFT), and based on this, a ring strain modulation system for cyclohexene was proposed to achieve the polymerization of cyclohexene

The ERSEs of the cyclohexene derivatives were investigated using density functional theory (DFT) at the B3YLP/6-31G(d,p) level of the theory (FIG. 1(b)). The calculated ERSE of 4,5-epoxy-1-cyclohexene (1, -0.66 kcal/mol) is less than that of cyclohexene (0.92 kcal/mol). 3,4-epoxy-1-cyclohexene (2, 1.62 kcal/mol) exhibits a slightly higher ERSE than cyclohexene. In particular, trans-4,5-cyclohexadiene carbonate (3, 6.00 kcal/mol) and trans-3,4-cyclohexadiene carbonate (5, 10.3 kcal/mol) exhibit significantly higher ERSEs than cyclohexene and may be suitable for ROMP. However, their cis isomers, cis-4,5-cyclohexadiene carbonate (4, 0.91 kcal/mol) and cis-3,4-cyclohexadiene carbonate (6, -0.90 kcal/mol), exhibit lower ERSEs than cyclohexene. The ERSEs of the neighboring cyclohexene-diols 6 (1.93 kcal/mol), 7 (2.13 kcal/mol), 8 (2.16 kcal/mol), and 9 (2.05 kcal/mol) are significantly lower than those of the trans carbonate-protected 3 and 5. This implies that in case of simple deprotection of the carbonate groups of 3 and 5, the metathesis reaction involving the monocyclic neighboring cyclohexene-diol derivatives favors the equilibrium for RCM over polymerization.

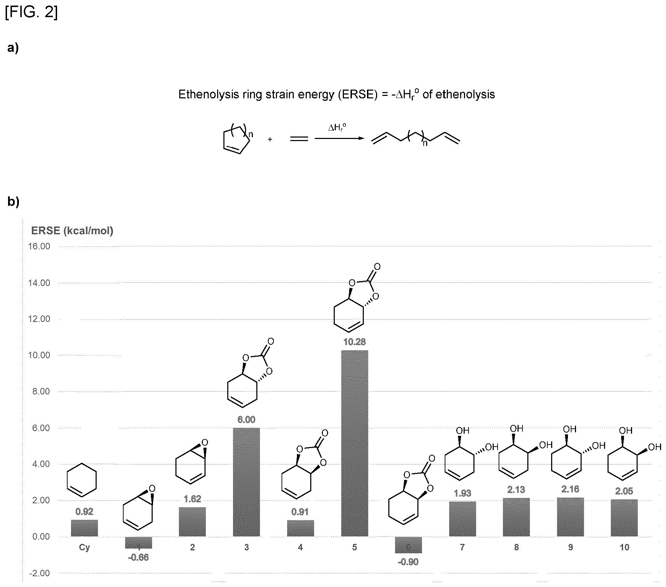

To understand how the fused ring increases the ring strain of cyclohexene, structural analysis of each monomer was performed. The structural data of monomers 3, 4 (Darensbourg, D. J. et al., Macromolecules 2014, 47, 7347-7353) and 5 (Darensbourg, D. J. et al., Macromolecules 2015, 48, 1679-1687) were obtained by single crystal X-ray diffraction and analyzed in comparison to other cyclohexene monomers obtained by DFT calculations. Newman projections of cis and trans cyclohexadiene carbonate were performed to investigate the effect of carbonate on ERSE (FIG. 2).

Newman projections of the cyclohexadiene carbonates on the bridged carbons showed that the changes in ring strain resulted from the fused ring structure. In trans-cyclohexadiene carbonates 3 and 5, the dihedral angles between the two CO bonds in the carbonate are 35.3° and 38.9°, respectively, which is unstable because the sp²-carbon-centered carbonate group prefers a planar structure. In trans-cyclohexadiene carbonate, the conformation of the cyclohexene ring remained a half-chair structure with a change in the bond angle involving the sp²-carbon. After the ethenolysis reaction to generate the open conformation, the dihedral angles between the C-O bonds of the cyclic carbonate groups decreased to 21.0° (3b) and 21.5° (5b), releasing the ring strain caused by the bicyclic structure.

For cis-cyclohexadiene carbonates, the dihedral angles between the two CO bonds in the ring-opened conformation after the ethenolysis reaction are similar or become larger (0.017° to 26.5° for 4 and 25.7° to 25.7° for 6). This is probably due to the increased steric hindrance of the open conformation induced by the alkyl chain on the same side. Since this destabilizing effect acts after the ring opening by ethenolysis, the ERSEs of the cis-cyclohexadiene carbonates 4 and 6 are smaller than those of the trans-isomers 3 and 5. Surprisingly, the ERSE of cis-1,4-cyclohexadiene carbonate (4, 0.91 kcal/mol) is higher than that of cis-1,3-cyclohexadiene carbonate (6, -0.90 kcal/mol), even though the dihedral angle difference between the two CO bonds is close to 0°. This is because the cyclohexene ring in 4a has a boat conformation to stabilize the cyclic carbonate, making 4a less stable than 6a, which has a semi-chair conformation, resulting in 4 having a higher ERSE value.

For monomers 1 and 2, both have small ERSE values (1, -0.66 kcal/mol; 2, 1.62 kcal/mol). The alkyl chains located on the same side in the cyclized forms 1b and 2b make the dihedral angle between the two alkyl chains very small (< 1°) due to the constrained structure by the epoxy group. The increased steric hindrance between the two alkyl groups in 1b and 2b causes 1 and 2 to have relatively low ERSEs. The ERSE is higher for 2 because of the conformation of the cyclohexene ring in 2a and the more mitigated steric hindrance in 2b. The conformation of the cyclohexene ring in 2 is a twisted boat structure, while the structure in 1 is a boat structure. It has been reported that the twisted boat is about 1.6 kcal/mol less stable than the boat conformation (Shishkin, O. V. et al., Practical Aspects of Computational Chemistry I: An Overview of the Last Two Decades and Current Trends, Leszczynski, J.; Shukla, M. K., Eds. Springer Netherlands: Dordrecht, 2012; pp 557-578). In addition, in the case of the alkyl chain in 2b, the allyl group has a mitigated steric hindrance due to its shorter chain length and more confined structure due to the 1,3-allylic strain.

### Example 2: Polymer synthesis and post-functionalization

The ROMP of the discussed oxygen-containing cyclohexene derivatives was carried out over the Grubbs third-generation catalyst (Ru1) (Love, J. A. et al., Angew. Chem. Int. Ed Engl. 2002, 41, 4035-4037), the Hoveyda-Grubbs second-generation catalyst (Ru2) (Kingsbury, J. S. et al., J. Am. Chem. Soc. 1999, 121, 791-799), the Grubbs second-generation catalyst (Ru3) (Scholl, M., Trnka, T. M., Morgan, J. P., Grubbs, R. H. Tetrahedron Lett. 1999, 40, 2247-2250), and the recently developed Ru4 catalyst, which has been reported to be highly active at low temperatures (Song, K. et al., Nat. Commun. 2019, 10, 3860).

Both 4,5-epoxycyclohexene (1) and the cis-carbonate fused cyclohexenes 4 and 6 have lower ERSEs than cyclohexene and did not undergo polymerization under the reaction conditions tested. The 1,2-diol functionalized cyclohexenes 7-10 have higher ERSEs than cyclohexene, but were unable to react due to precipitation at low temperature conditions due to their low solubility, and did not undergo polymerization at 20°C. However, 3,4-epoxycyclohexene 2, with an ERSE of 1.62 kcal/mol, was reactive at low temperature conditions with Ru4 catalyst (9.5% conversion at -40°C and 21% conversion at -60°C; Table 1, entries 4 and 5). Gel permeation chromatography (GPC) indicated that the resulting P2 polymers had a number average molecular weight (Mₙ) of 6.70 and 9.72 kDa, respectively, demonstrating that polymers were indeed generated.

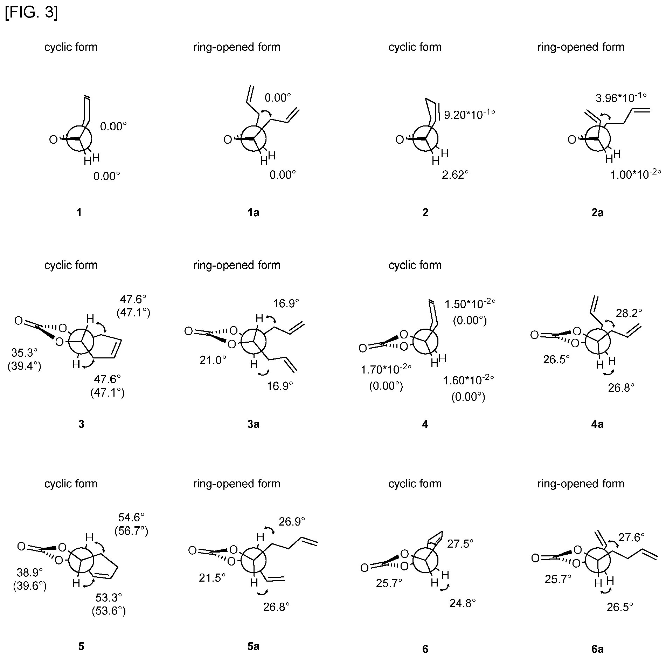

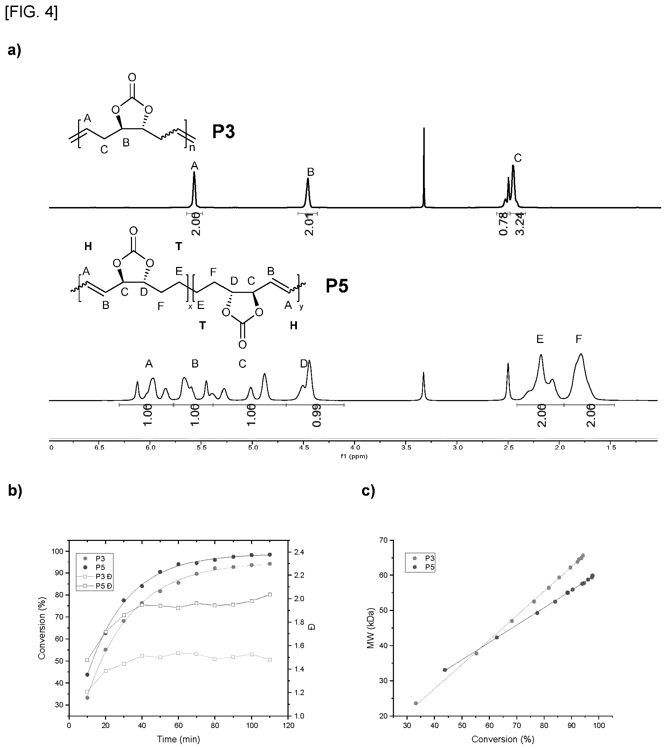

Based on the results, ROMP of trans-carbonate-fused cyclohexenes 3 and 5 with high ERSE was attempted. The ROMP proceeded smoothly for both monomers with high conversion (> 95%) using Ru1 within 30 min at 20°C. However, polymerization was too fast at 20°C, so for convenience, kinetic experiments were performed at 10°C to reduce the reaction rate. In the kinetic experiments, the polymerization exhibited typical primary kinetics (FIG. 3(b)) and the molecular weight showed linear growth with conversion (FIG. 4(c)). The linear growth of the polymer molecular weight through the reaction and the dispersion retained after 20 min indicate that controlled polymerization occurs during the ROMP of the trans-carbonate monomer. The reactions were then carried out under the same conditions using different monomer to initiator (M-to-I) ratios (Table 1, entries 7-16). When Mₙ of P3 and P5 is plotted as a function of M-to-I ratio (FIG. 4(c)), it shows a linearly increasing Mₙ (r² = 0.99, red line), indicating a well-controlled ROMP. Polymers with narrow dispersion values (≤1.3) were obtained at high M-to-I ratios, e.g., at an M-to-I ratio of 1000:1, with P5 exhibiting Mₙ of 122.7 kDa and Ð of 1.30, and P3 exhibiting Mₙ of 145.5 kDa and Ð of 1.19.

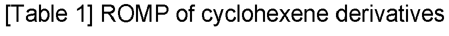

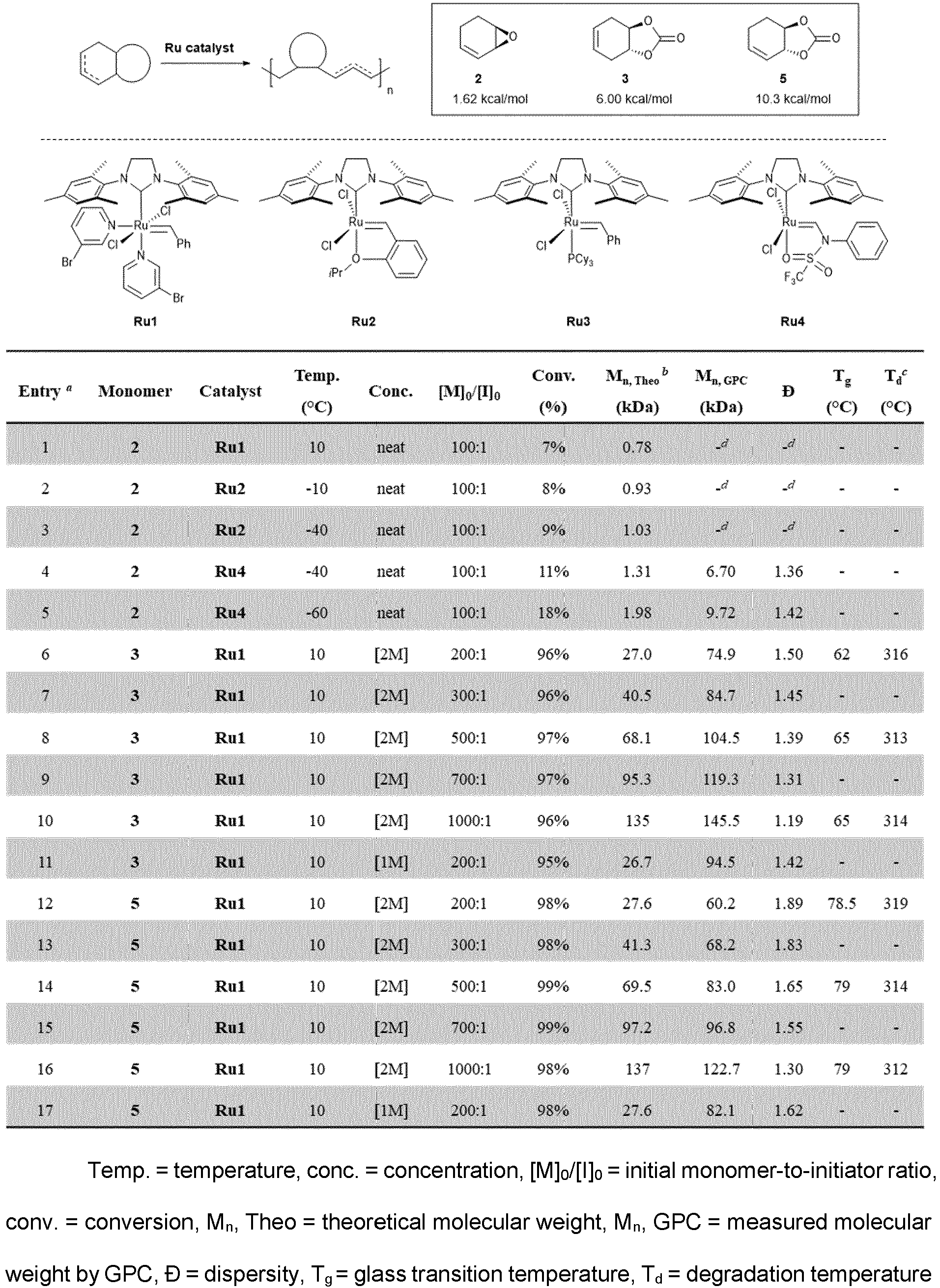

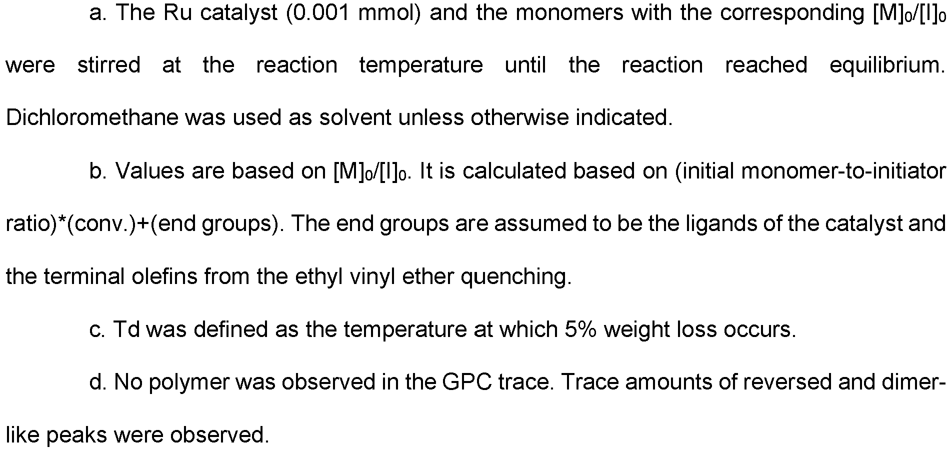

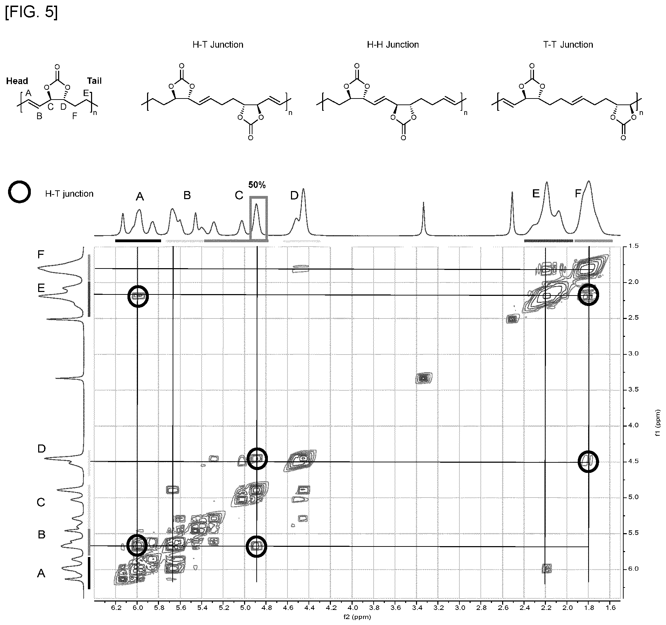

Since monomer 3 is symmetrical, P3 exhibits a very regular structure that can be observed in its nuclear magnetic resonance (NMR) spectrum (FIG. 4(a)). However, the olefin peak of P3 at 5.58 ppm has a slight shoulder peak at 5.57 ppm, and the ¹³C NMR of P3 shows two peaks (127.75 and 126.58 ppm) in the *sp²* carbon region, wherein the polymer has a mixture of *cis-* and *trans*-olefins. The ratio of *cis-* and *trans*-olefins was observed by inverse-gated decoupling ¹³C NMR spectroscopy with an elongated relaxation delay, and the trans-olefin is dominant (comparing the area between the two *sp²* carbon peaks), accounting for about 78%. In addition, the 3 used in the reaction is a racemic mixture, so the resulting P3 is atactic. For monomer 5, which has an asymmetric structure, P5 showed poor positional regularity despite having an allyl substituent (Kobayashi, S. et al., J. Am. Chem. Soc. 2011, 133, 5794-5797; Brits, S. et al. Chem. Lett. 2018, 9, 1719-1727; Guillory, G. A. et al. J. 2019, 120, 109251; Liu, F. et al. J. 2020, 124, 109472). In the HSQC spectra, the signals of proton C and D in the carbonate ring clearly show that the polymer has three different conjugations: head-to-tail (HT), head-to-head (HH), and tail-to-tail (TT). The peak of the HT conjugation identified in the COSY spectrum accounts for about 50% of the proton C (~4.88 ppm), indicating that the monomer was added to the polymer chain completely randomly (FIG. 5). Since the positional selectivity of an allyl substituent is strongly influenced by the volume of that substituent, the carbonate ring restricting the movement of the allyl substituent in monomer 5 may have contributed to the low positional selectivity of P5.

Deprotection of the carbonate units was performed for the modification of the polymer repeating units from the highly modified trans-carbonate protected form to the less modified diol form. Upon addition of an excess of lithium hydroxide at room temperature, quantitative deprotection of the carbonate groups was observed from IR and NMR spectra (FIG. 64 and FIG. 65).

Since ROMP polymers are often used after hydrogenation to obtain functionalized polyethylene with better stability and optical performance, hydrogenation of the polymer was also performed. The hydrogenation reaction was carried out in an autoclave reactor filled with hydrogen gas and RuCl₂(PPh₃)₃ was used as a catalyst (Rao, P. V. C. et al., Eur. Polym. J. 2001, 37, 1159-1164). During the hydrogenation reaction, quantitative hydrogenation of the olefin was observed, while the carbonate group remained unaffected, resulting in producing the hydrogenated polymers, P_{H}3 and P_{H}5 (FIGs. 64 and 65). After hydrogenation, 1,2-diol polymers P_{HD}3 and P_{HD}5 were obtained from deprotection of the carbonate group (FIGs. 64 and 65).

The deprotected polymers (P_{D}3, P_{D}5, P_{HD}3, and P_{HD}5) form well-defined polyvinyl alcohol (PVA) copolymers with a structure that has one trans-1,2-diol in all six carbons of the backbone. These deprotected 1,2-diol polymers have a structure similar to ethylene vinyl alcohol copolymers (EVOH), which are widely used as packaging materials due to their excellent oxygen and hydrocarbon barrier properties, and have high potential in a variety of applications (Yeh, J.-T. et al., J. Mater. Sci. 2001, 36, 1891-1900; Lagaron, J. M. et al. Test. 2001, 20, 569-577; Han, X. et al., Desalination 2009, 240, 21-26; Minelli, M. et al., Polym. Eng. Sci. 2010, 50, 144-153; López-Rubio, A. et al., In Multifunctional and Nanoreinforced Polymers for Food Packaging, Lagarón, J.-M., Ed. Woodhead Publishing: 2011; pp 261-284).

### Example 3: Measuring thermal and mechanical properties

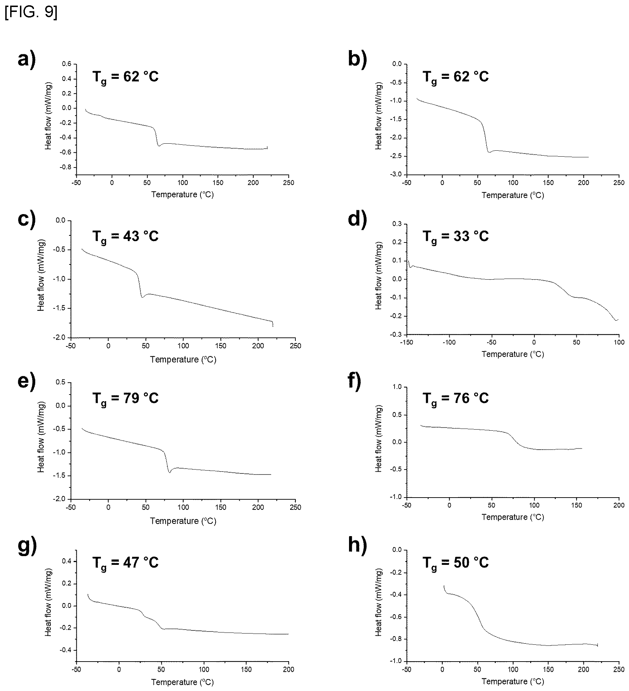

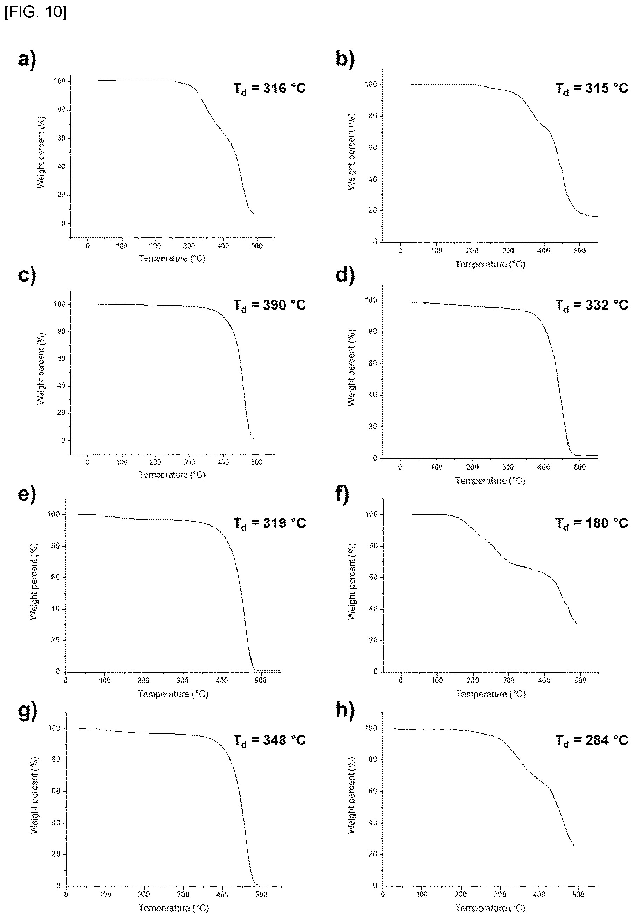

The glass transition temperatures (T_{g}) of the purified and fully dried polymer samples were measured by differential scanning calorimetry (DSC) on a DSC Q20, TA instrument. All T_{g} values were obtained from the second or third scan after removing the thermal history by the first scan. The warming rate was 10°C/min. The decomposition temperature (T_{d}) of the polymer samples was measured by thermogravimetric analysis (TGA) on a TGA Q50, TA instrument. The value of T_{d} was defined as the point at which 5% mass loss occurred. The heating rate was 10°C/min. The results are shown in FIGs. 9 and 10.

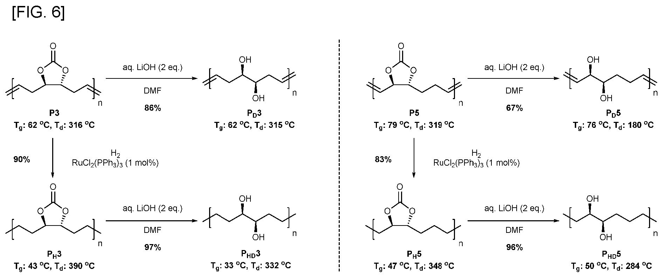

The thermal properties of the obtained polymers were obtained from thermogravimetric analysis (TGA) and differential scanning calorimetry (DSC) (FIG. 6, FIG. 9 and FIG. 10). The carbonate polymers (P3, P5, P_{H}3, and P_{H}5) showed high thermal stability of at least 300°C. After hydrogenation, the T_{d} of P_{H}3 and P_{H}5 were 390°C and 348°C, respectively, which were higher than the T_{d} of P3 and P5, 316 °C and 319 °C, showing higher thermal stability. However, the hydrogenated polymers showed lower glass transition temperatures (T_{g}) of 43 °C and 47 °C, while the T_{g} of P3 and P5 were 62°C and 79°C. The same trend was observed for the 1,2-diol polymers. P_{D}3 and P_{D}5 have higher T_{g} and lower T_{d} compared to hydrogenated polymers.

The obtained polymers showed high resistance to common organic solvents, similar to carbonate-protected cyclooctenediol polymers (Scherman, O. A. et al., Macromolecules 2005, 38, 9009-9014). Polymers P3, P5, P_{D}3, P_{D}5, P_{H}3, and P_{H}5 could be dissolved only in highly polar organic solvents (DMF, DMSO, NMP), while P_{HD}3 and P_{HD}5 were insoluble in organic solvents and water.

Polymers P3, P5, P_{D}3, P_{D}5, P_{H}3, and P_{H}5 were dissolved in anhydrous DMF at a concentration of 100-200% w/v and then deposited by bar coating at a specific rate (10 mm/s) on a glass or silicon substrate to produce polymer films of various thicknesses. The films were dried in a vacuum oven at 35°C for 24 hours and then in a vacuum oven at 65°C for 24 hours to remove any remaining solvent. P_{HD}3 and P_{HD}5 were also prepared by hot-pressing under conditions of 110°C and 100 bar. The mechanical properties and oxygen and water permeability of the polymer films were subsequently measured.

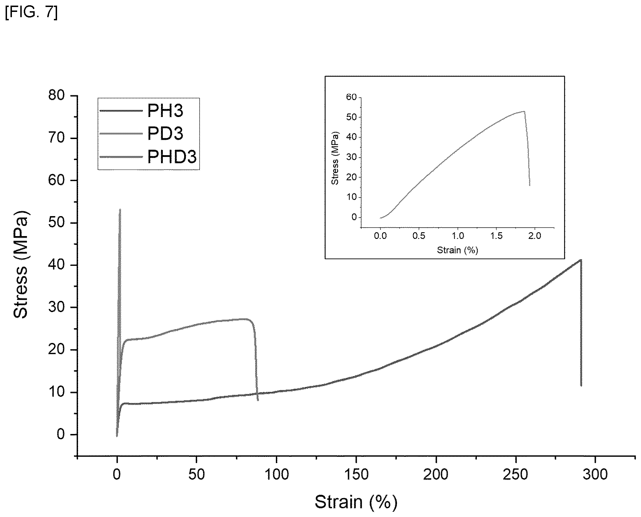

The mechanical properties of P_{H}3, P_{D}3, and P_{HD}3 films were measured. (FIG. 7) The tensile strength of polymer film samples in ASTM D882 form was measured by UTM at 23°C, 45% relative humidity, at a speed of 50 mm/min and a grip length of 40 mm, with values averaged over at least three measurements. P_{H}3 film (40 µm thick) had a tensile strength of 41.2 MPa (approximately 290% elongation at break). The Young's modulus was 3.19 GPa and the yield point was observed at 4.56% strain and 6.75 MPa. P_{D}3 film (30 µm thick) had a tensile strength of 49.0 MPa (about 2% elongation at break). The Young's modulus was 26.4 GPa, and the yield point was observed at 1.93% strain, 48.9 MPa. P_{HD}3 film (110 µm thick) had a tensile strength of 27.7 MPa (about 95% elongation at break). The Young's modulus was 9.44 GPa and the yield point was found at 5.3% strain and 21.3 MPa.

### Example 4: Hydrolytic stability of PVA copolymers

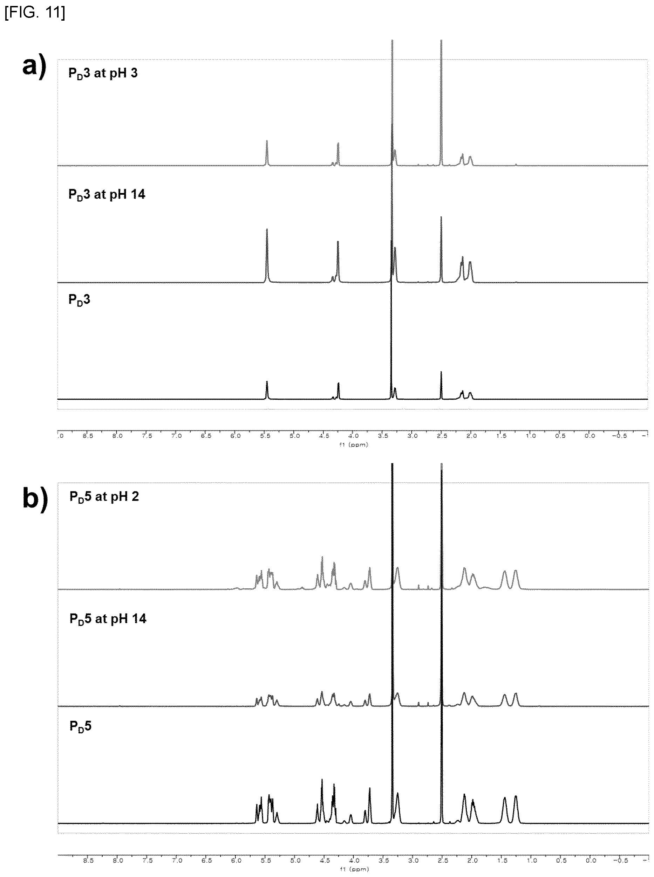

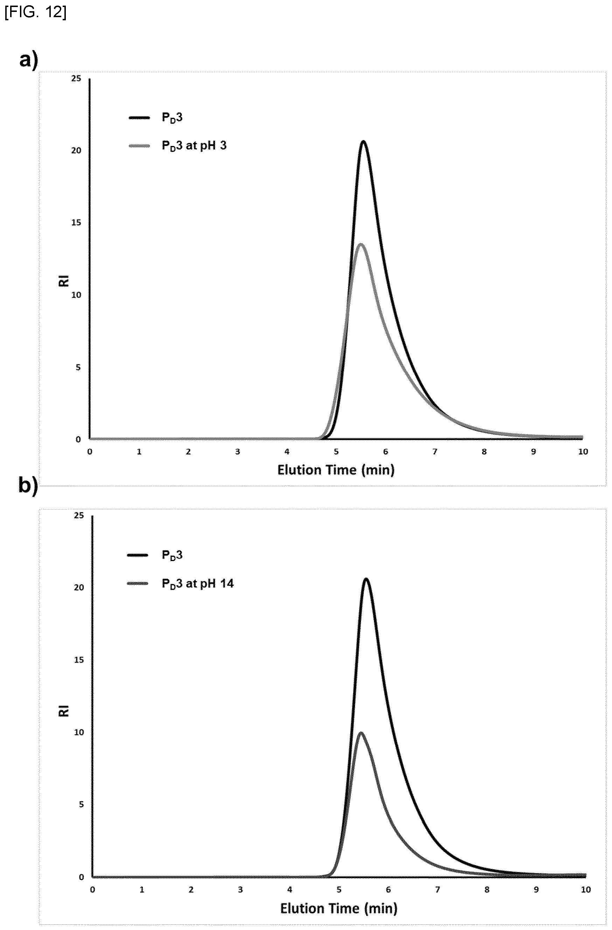

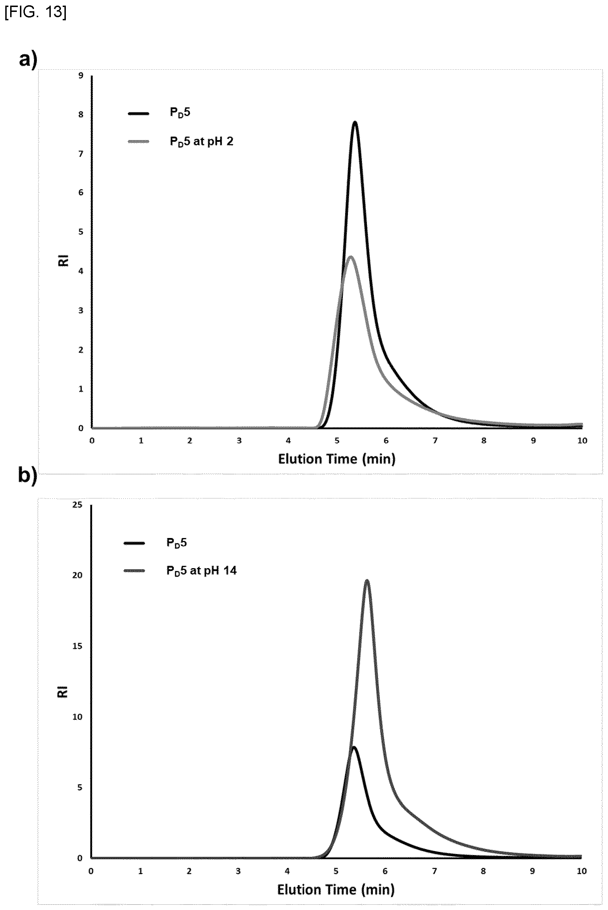

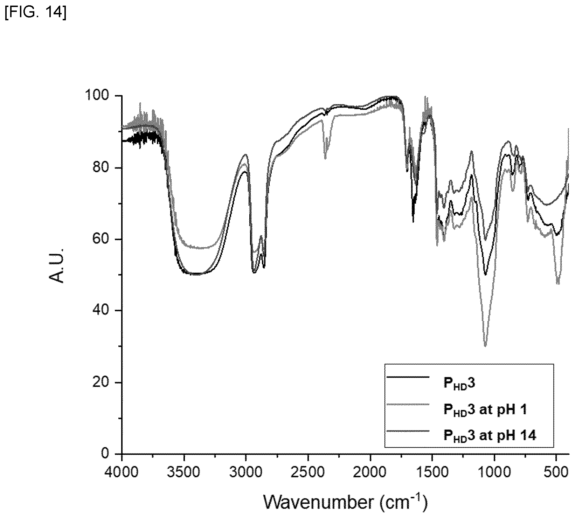

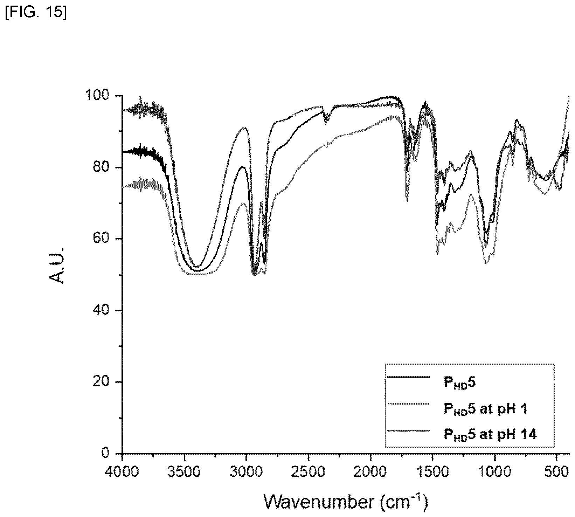

The hydrolytic stability of the 1,2-diol polymers P_{D}3 and P_{D}5, and P_{HD}3 and P_{HD}5 was determined by stirring the polymer samples in aqueous solutions of HCl or NaOH with a specific pH for 18 hours. The stability of the polymers was measured by NMR spectroscopy and GPC tracing for P_{D}3, P_{D}5, and by IR spectroscopy for P_{HD}3, P_{HD}5 due to the solubility issues. The results are shown in FIGs. 11 to 15.

### Example 5: Oxygen and water vapor permeability measurement

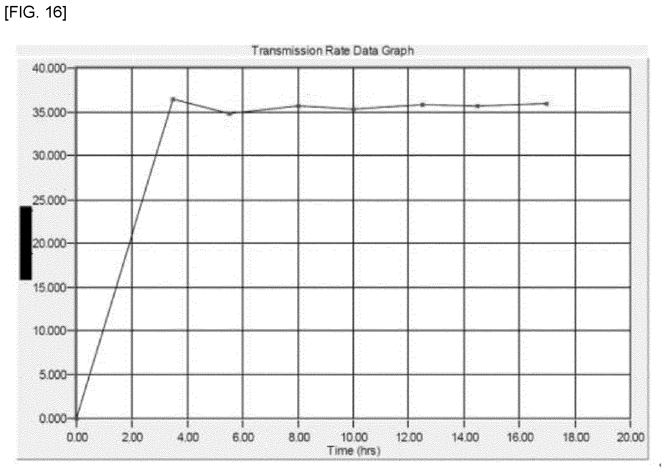

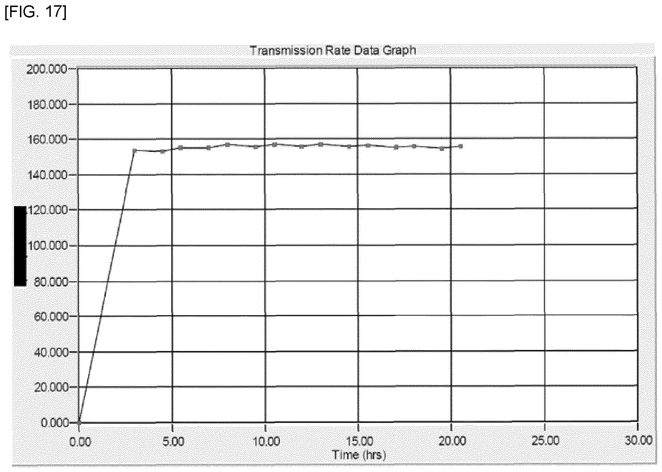

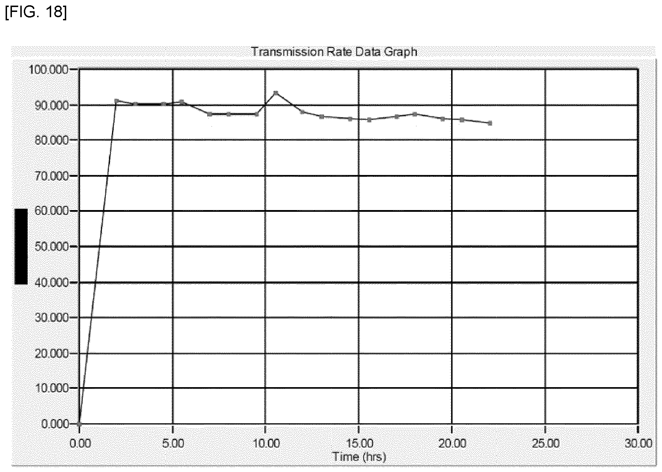

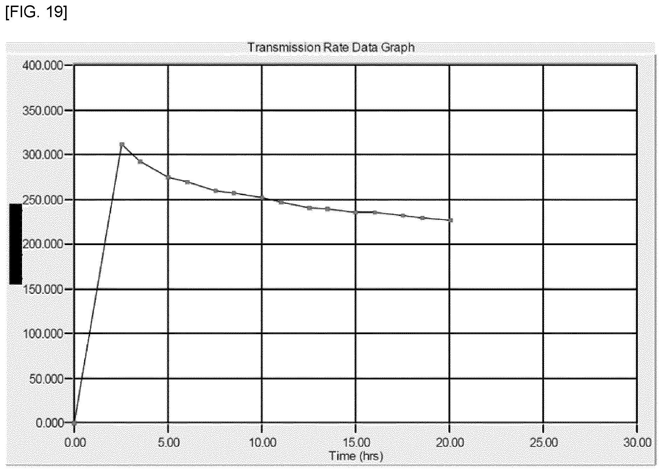

The oxygen transmission rate (OTR) of the polymer films was measured according to ASTM D3985 using an OX-TRAN Model 2/21, Mocon. Water vapor transmission rate (WVTR) was measured according to ASTM F1249 using Permatran-W 3/33 MA, Mocon. The results are shown in FIGs. 16 to 27.

The oxygen and water vapor permeability of the obtained films was measured according to ASTM-D3985 and ASTM-F1249, and the results were averaged over two experiments with an average film thickness of 100 µm. The large difference between T_{g} and T_{d} of the polymers allows the polymers to be easily processed over a wide temperature range. The films of P3, P5, P_{H}3, P_{H}5, P_{D}3, and P_{D}5 were solution cast in silicone molds in DMF solution and dried at 60°C for 48 hours in a vacuum oven to produce transparent films. P_{HD}3 and P_{HD}5 were hot-pressed at 110°C, and 100 bar to produce transparent films. The oxygen transmission rate (OTR) and water vapor transmission rate (WVTR) of the films were 35.9 cm³/(m²·24h·atm), 155 g/(m²·24h·atm) for P3, 84.7 cm³/(m²·24h·atm), 226 g/(m²·24h·atm) for P5, 61.9 cm³/(m²·24h·atm), 119 g/(m²·24h·atm) for P_{H}3, 72.9 cm³/(m²·24h·atm), 138 g/(m²·24h·atm) for P_{D}3, 1.69 cm³/(m²·24h·atm), >500 g/(m²·24h·atm) for P_{D}5, 2.50 cm³/(m²·24h·atm), >500 g/(m²·24h·atm), and 3.03 cm³/(m²·24h·atm), 349 g/(m²·24h·atm) for P_{HD}3 (FIG. 16 to FIG. 27). Compared to a pure PVA film with an OTR of 5.96 cm³/(m²·24h·atm) and a WVTR of 790-1400 g/(m²·24h·atm), P_{HD}3 showed water resistance and improved oxygen barrier properties.

### Example 6: Polymer recycling

Among the 1,2-diol polymers, P_{D}3 and P_{D}5 are composed of ring-opening repeating units of monomers 7 and 9 with low ERSE values of 1.93 and 2.16 kcal/mol, respectively, allowing the recycling of these 1,2-diol polymers.

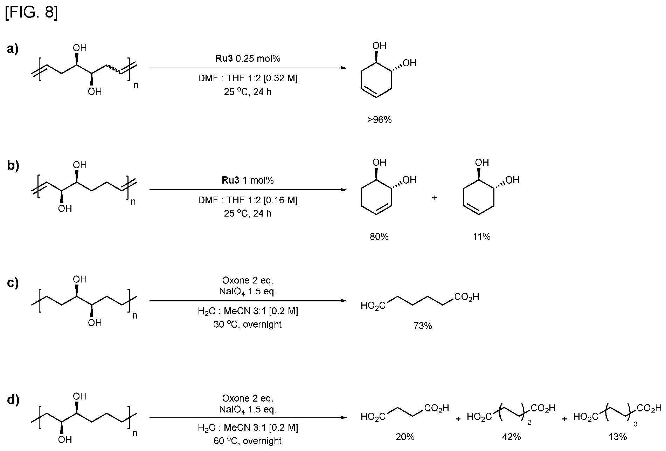

The 1,2-diol polymers P_{D}3 and P_{D}5 are composed of ring-opening repeating units of compounds 7 and 9 with low ERSEs of 1.93 and 2.16 kcal/mol, respectively, indicating that the RCMD reactions of P_{D}3 and P_{D}5 are thermodynamically favored (see FIG. 8, Table 2). P_{D}3 decomposed at 25°C in a co-solvent containing DMF THF = 1:2, 0.25 mol% Ru3, 0.32 M olefin concentration to give 7 in almost quantitative yield (> 96%) after 24 hours. The RCMD of P_{D}5 proceeded smoothly and gave 9 in very good yield (80%) after 24 hours at 25°C in a co-solvent containing DMF:THF = 1:2 at a concentration of 1 mol% Ru3, 0.16 M olefin in very good yield (80%). The obtained vicinal trans-cyclohexene-diols 7 and 9 could be recycled to monomers 3 and 5, respectively, by carbonate protection to complete the closed-loop recycling. Compounds 7 and 9 are also versatile chemical feedstocks for the synthesis of 3- and 2-hexene derivatives, bisphenols, and diamines.

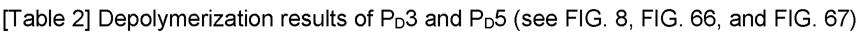

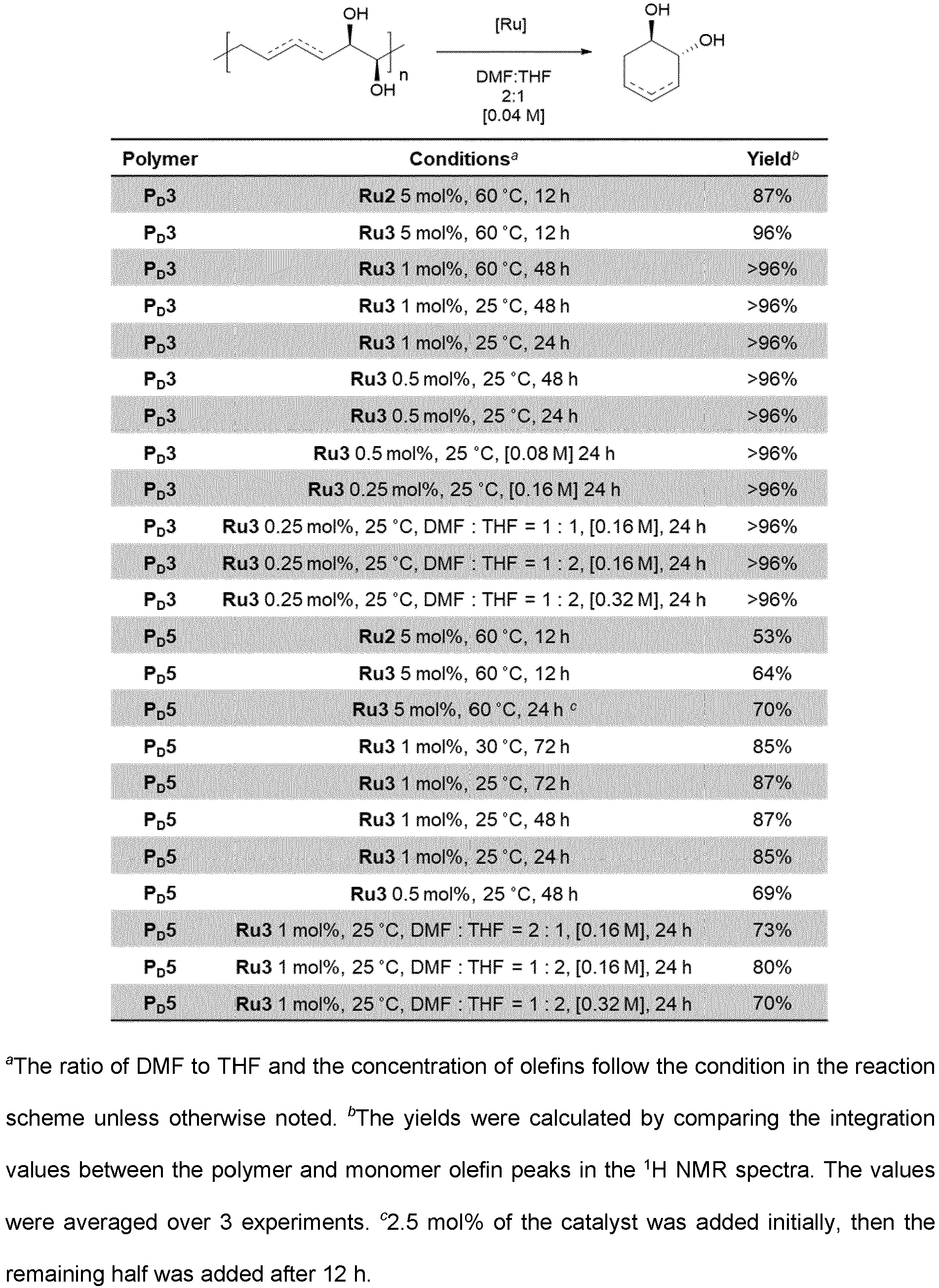

### Chemical recycling of polymers into α,ω-dialdehyde and α,ω-dicarboxylic acid

Recyclable ROMP polymers with open loops have been actively developed by designing engineered monomers containing chemically cleavable functional groups. Grubbs and coworkers reported acetal-loaded ROMP polymers prepared by copolymerization of cyclooctene with 4,7-dihydro-1,3-dioxepine, which can be hydrolyzed under both acidic (10% HCl in MeOH) and basic conditions (0.7 M NaOMe in MeOH) and recycled into hydroxy telechelic polymers (Fraser, C. et al, Macromolecules 1995 28, 7256-7261). Schlaad et al. also reported acid-hydrolyzable acetal-incorporated ROMP polymers prepared from biomass-derived levoglucosanol (Debsharma, T. et al. Angew. Chem. Int. Ed Engl. 2019, 58, 6718-6721; Debsharma, T. et al., Macromolecules 2021, 54, 2720-2728). Fishman and Kiesling reported that heterocyclic oxazinone-based ROMP monomers can be degraded under both acidic (pH < 4.5) and basic conditions (pH > 9) (Fishman, J.M. et al., Angew. Chem. Int. Ed Engl. 2013, 52, 5061-5064). Xia and co-workers reported the preparation of ROMP polymers from the cyclic enol ether, and 2,3-dihydrofuran, which are degraded rapidly in dilute HCl solution (~20 mM) (Feist, J.D. et al., J. Am. Chem. Soc. 2020, 142, 1186-1189; Feist, J.D. et al. 2021, 14, 53-58). Johnson and co-workers reported several linear and crosslinked ROMP polymers based on silyl ether monomers that are susceptible to degradation in the presence of acids or TBAF (Shieh, P. et al., Nat. Chem. 2019, 11, 1124-1132; Shieh, P. et al., Nature 2020, 583, 542-547; Husted, K.E.L. et al., ACS Macro Lett. 2021, 10, 805-810). Aminal (Animal, Bhaumik, A. et al., J. Am. Chem. Soc. 2019, 141, 12207-12211), phosphodiester (Steinbach, T. et al., Polym. Chem. 2013, 4, 3800-3806; Chang, C.-C., et al., Macromolecules 2014, 47, 1344-1350), disulfide (Chang, C.-C., et al., Macromolecules 2014, 47, 1344-1350), and other functional groups (Mallick, A., et al., Polym. Chem. 2018, 9, 372-377; Haider, T. et al., Macromolecules 2019, 52, 2411-2420) have also been used to develop chemically recyclable polymers. However, the use of hydrolyzable groups as the main recycling moiety can lead to poor hydrolytic stability in practical applications.

1,2-diol polymers can be cleaved by the well-known oxidative cleavage reaction of vicinal diols. Unlike other common degradable ROMP polymers, they have a high hydrolytic stability due to the fact that their polymer backbone consists exclusively of hydrocarbons. Furthermore, the cleavage of 1,2-diol polymers yields α,ω-dialdehyde or α,ω-dicarboxylic acid as products, which can be useful feedstocks for a variety of organic reactions. Indeed, the 1,2-diol polymers P_{D}3, P_{D}5, P_{HD}3, and P_{HD}5 exhibited high resistance to acidic and basic conditions (FIGs. 11, 12, 13, 14, and 15). To test hydrolytic stability, the polymers were stirred for 18 hours at room temperature in aqueous solutions of HCl or NaOH at a specific pH. After exposure to acidic or basic conditions, the polymer structure was analyzed using ¹H NMR, GPC, or IR spectroscopy. P_{D}3 exhibits high resistance to basic conditions up to pH of 14. Under acidic conditions, P_{D}3 is stable up to pH of 3. However, below pH 3, cross-linking of the polymer chains is observed. P_{D}5 is stable in basic conditions up to pH of 14 and in acidic conditions up to pH of 2. In other words, it exhibits better stability than P_{D}3 in acidic conditions. Both P_{HD}3 and P_{HD}5 exhibit high hydrolytic stability under acidic and basic conditions and retain their structure at pH of 1 and pH of 14.

The oxidative degradation of C-C bonds to generate dioxide is well used in traditional organic synthesis and in the upcycling of polymers (Bäckström, E. et al. Eng. Chem. Res. 2017, 56, 14814-14821; Criegee, R. Ber. dtsch. Chem. Ges. A/B 1931, 64, 260-266; Wang, Z. (2010). Malaprade Reaction. Comprehensive Organic Name Reactions and Reagents. John Wiley & Sons, Inc.; Amadio, E. et al. 2018, 557, 89-98; Sato, K. et al., Science 1998, 281, 1646-1647; Spannring, P. et al., RSC Adv. 2013, 3, 6606-6613). Based on the fact that the degradation of the developed 1,2-diol polymers can yield industrially useful α,ω-dicarboxylic acids, the efficient degradation conditions for P_{HD}3 and P_{HD}5 were investigated under various 1,2-diol degradation conditions. Conditions containing sodium periodate, RuCl₃ catalyst and sodium periodate, oxone (2 equivalents) and sodium periodate (1.5 equivalents) were applied to the oxidative degradation reactions.

### Oxidation with sodium periodate

Degradation studies of 1,2-diol polymers were carried out in a CD₃CN/D₂O co-solvent system using two equivalents of sodium periodate. After degradation, P_{HD}3 gave hexane-1,6-dialdehyde as the single major product and P_{D}3 gave 2,4-hexadiene-1,6-dialdehyde (muconaldehyde) as the single major product, as they both have symmetrical repeating units. In contrast, P_{HD}5 and P_{D}5 gave three major products of different lengths at the HH, HT, and TT conjugation. These dialdehydes can be upcycled into useful feedstocks for polymer synthesis and various organic reactions. Muconaldehyde has been used in the synthesis of heterocycles (Bleasdale, C. et al., Chem. Res. Toxicol. 1993, 6, 407-412), the Diels-Alder reaction (Epiotis, N. D. et al., J. Org. Chem. 1974, 39, 3150-3153), a linking substrate in polyenes and biosystems (Markl, G. A. et al., Helv. Chim. Acta 2003, 86, 2589-2609; Craig, G. S. W. et al., Macromolecules 1995, 28, 2512-2518; Vosburg, D. A. et al., Chirality 2003, 15, 156-166; Rivedal, E. et al. Biol. Interact. 2010, 184, 229-32; Latriano, L. et al. Health Perspect. 1989, 82, 249-251). Furthermore, functionalized polyamides and poly(silyl ethers) can be synthesized from α,ω-dialdehyde (Wang, Y.-Z. et al., Polym. Chem. 2013, 4, 444-448; Vijjamarri, S. et al. Chem. Eng. 2018, 6, 2491-2497). Finally, α,ω-dialdehydes can undergo simple transformations by oxidation or reduction to produce α,ω-dicarboxylic acids and α,ω-diols. These α,ω-dicarboxylic acids and α,ω-diols are widely used in various organic reactions and are used as raw materials for the synthesis of polyester, polyurethane, and polyamide.

### Oxidation with a RuCl₃ catalyst

The 1,2-diol polymer, RuCl₃·H₂O (0.02 equivalents per 1,2-diol) and NaIO₄ (4 equivalents per 1,2-diol) were added to CH₃CN/H₂O/EtOAc co-solvent (0.13 M). In a reaction vial in a ratio of 3:2:2. The mixture was sonicated for 2 hours and then acidified with an excess of HCl. The crude product was extracted three times with EtOAc. The combined organic phases were concentrated in vacuo. The crude extract was analyzed by gas chromatography using 10 µL dodecane as an internal standard (P_{HD}5) or by ¹H NMR using dimethyl sulfone as an internal standard (P_{HD}3).

This oxidative degradation reaction degraded P_{HD}3 to give adipic acid (83%) 3, an essential feedstock for nylon-6 (Table 3). Under the same conditions, P_{HD}5 gave a mixture of dicarboxylic acids containing succinic acid (4C), adipic acid (6C), and suberic acid (8C) in 78% yield, with a 4C:6C:8C ratio of 1:5.2:1.2 (Table 3). This may be due to the addition selectivity issues described in the synthesis of P5.

### Oxidation with oxone

The 1,2-diol polymer, oxone (2 eq. per 1,2-diol) and NalO₄ (1.5 eq. per 1,2-diol) were added to CH₃CN/H₂O co-solvent (0.2 M) in a reaction vial. In a ratio of 1:3. the mixture was stirred at reaction temperature overnight. The mixture was extracted three times with diethyl ether. The organic phase was dried with Na₂SO₄, filtered and concentrated. The crude product was analyzed by gas chromatography using 10 µL dodecane as an internal standard (P_{HD}5) or by ¹H NMR using dimethyl sulfone as an internal standard (P_{HD}3).

Under the condition of using oxone (potassium peroxymonosulfate [KHSO₅·0.5 KHSO₄·0.5 K₂SO₄)] as an oxidizing agent, 73% adipic acid was obtained as a decomposition product of P_{HD}3, while for P_{HD}5, a 75% discrete mixture was obtained with a 4C:6C:8C ratio of 1.5:3.5:1 (Table 3). This may be due to the addition selectivity problem described in the synthesis of P5. The obtained α,ω-dicarboxylic acids are key feedstocks for the synthesis of polyesters, polyurethanes, and polyamides, and various organic reactions. Compared to the generation of carbon dioxide and water when commercial PVA is fully degraded, the developed polymers, which produce these useful substances after degradation, will contribute to the reduction of polymer waste and conservation of limited resources.

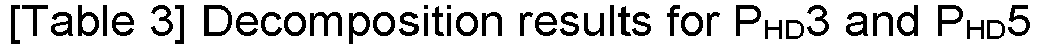

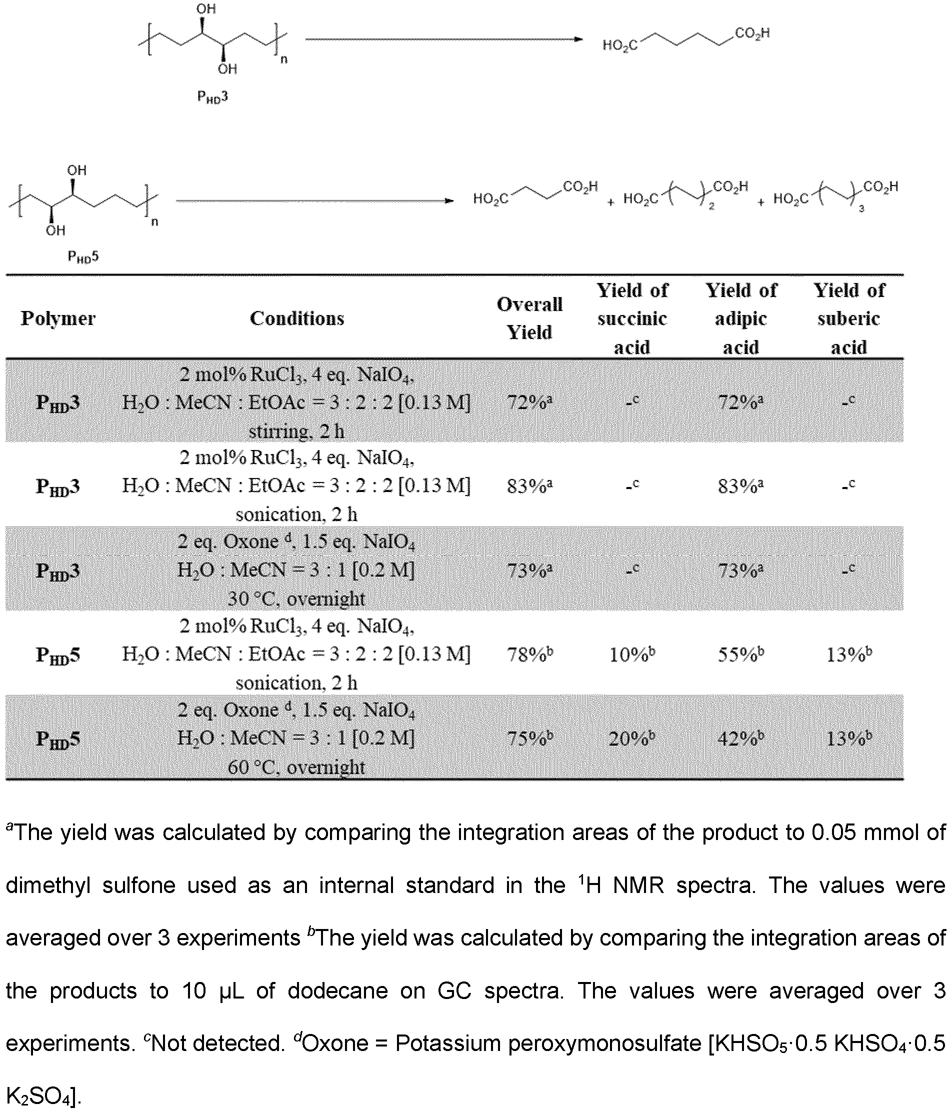

In conclusion, the first homopolymerization of cyclohexene derivatives was achieved by the control of the monomer ring strain through the designed monomers. The polymerization of the monomers was predicted by the calculation of ERSE; in particular, the trans-carbonate unit is effective in modifying the structure of cyclohexene to increase the RSE. Trans-3,4-cyclohexadiene carbonate (ERSE: 10.3 kcal/mol) and trans-4,5-cyclohexadiene carbonate (ERSE: 6.00 kcal/mol) both exhibited high conversion and controlled ROMP under room temperature conditions. The subsequent 1,2-diol polymer obtained after deprotection of the carbonate from the resulting polymer could be chemically recycled to the monomers cyclohexene-3,4-diol and cyclohexene-4,5-diol by a facile RCMD reaction with low ERSEs of 2.16 and 1.93 kcal/mol. Furthermore, the 1,2-diol-polymer can be upcycled to α,ω-dialdehyde and dicarboxylic acid by oxidative cleavage of the 1,2-diol on the polymer skeleton, which can be used as feedstock for polymers and various organic reactions. The present invention demonstrates that novel chemically recyclable polymers with high oxygen barrier properties and hydrolytic stability can be developed by molecular design of versatile monomers. This new PVA copolymer, if used in industry, can significantly reduce the amount of packaging waste and save resources.

While the foregoing has described in detail certain aspects of the present invention, it is apparent to one of ordinary skill in the art that these specific descriptions are merely preferred embodiments and are not intended to limit the scope of the present invention. Accordingly, the substantial scope of the present invention will be defined by the claims and their equivalents.

## Claims

1. A method of preparing a polyvinyl alcohol copolymer, comprising:
(a) subjecting a cyclohexene compound with a carbonate protecting group to a ring-opening metathesis polymerization (ROMP) in the presence of a catalyst to obtain a polycarbonate of Formula 1; and
(b) subjecting the compound of the Formula 1 to carbonate deprotection to obtain a polyvinylalcohol copolymer of Formula 4. 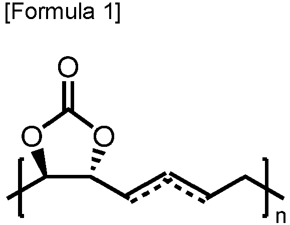 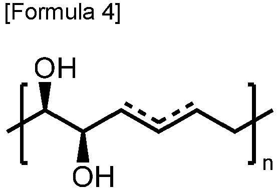 wherein n is an integer of 1 to 100,000.

2. A method of preparing a polyvinyl alcohol copolymer, comprising:
(a) subjecting a cyclohexene compound with a carbonate protecting group to ring-opening metathesis polymerization (ROMP) in the presence of a catalyst to obtain a polycarbonate of Formula 1;
(b) performing a hydrogenation reaction of the polycarbonate of Formula 1 in the presence of a catalyst to obtain a polycarbonate of Formula 2; and
(c) subjecting the polycarbonate of Formula 2 to carbonate deprotection to obtain a polyvinylalcohol copolymer of Formula 5. 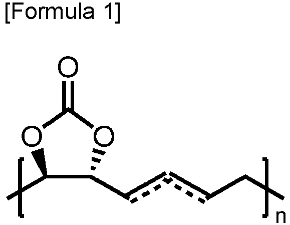 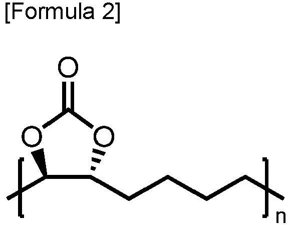 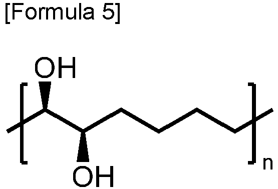 wherein n is an integer of 1 to 100,000.

3. The method of preparing a polyvinylalcohol copolymer of claim 1 or 2, wherein the cyclohexene compound is obtained by converting a 1,2-diol group from a cyclohexene diol to a carbonate group.

4. The method of preparing a polyvinylalcohol copolymer of claim 1 or 2, wherein ethenolysis ring-strain energy of the cyclohexene compound is 1 to 30 kcal/mol.

5. The method of preparing a polyvinylalcohol copolymer of claim 1 or 2, wherein the cyclohexene compound with a carbonate protecting group is represented by Formula 3:

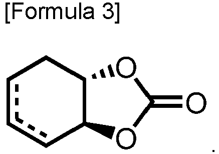

6. The method of preparing a polyvinylalcohol copolymer of claim 1 or 2, wherein the catalyst in the ring-opening metathesis polymerization (ROMP) is selected from the following formulae:

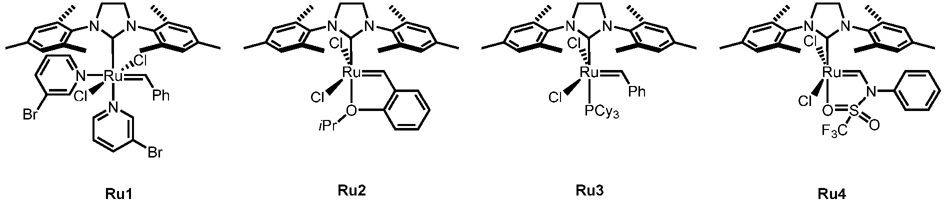

7. The method of preparing a polyvinylalcohol copolymer of claim 1 or 2, wherein the cyclohexene compound is a trans-4,5-cyclohexadiene carbonate of Formula 3-1 or a trans-3,4-cyclohexadiene carbonate of Formula 3-2.

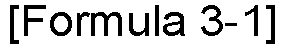

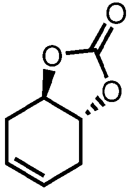

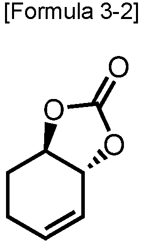

8. The method of preparing a polyvinylalcohol copolymer of claim 2, wherein the catalyst in the hydrogenation reaction is selected from the group consisting of RuCl₂(PPh₃)₃, Pd/C, ruthenium pincer complexes, rhodium catalysts, TsNHNH₂, and hydrogen (H₂).

9. A polyvinylalcohol copolymer, represented by Formula 4 or Formula 5: 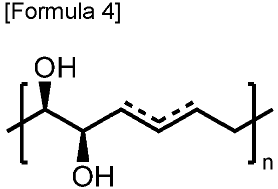 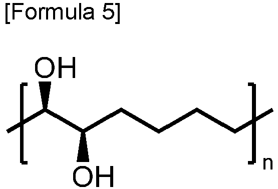 wherein n is an integer of 1 to 100,000.

10. A method for recycling a cyclohexene monomer, comprising:
(a) subjecting polyvinyl alcohol copolymer of Formula 4 to a ring-closing metathesis (RCM) to obtain a compound of Formula 6; and
(b) subjecting the compound of Formula 6 to carbonate protection to obtain a cyclohexene monomer. 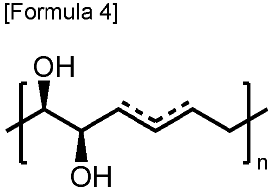 wherein n is an integer of 1 to 100,000.

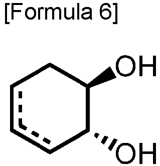

11. A method of producing a useful organic material, comprising performing a diol oxidation reaction of a polyvinyl alcohol copolymer of Formula 5 to obtain a carboxylic acid of Formula 7, an aldehyde of Formula 8, or an alcohol of Formula 9: 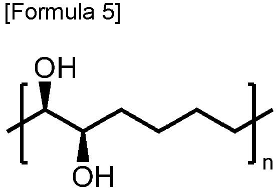 wherein n is an integer of 1 to 100,000.

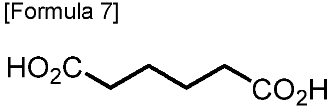

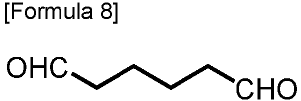

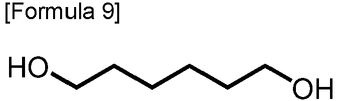

12. A polymeric electrolyte comprising the polyvinyl alcohol copolymers of claim 9.
